# FASCICULE DE BREVET EUROPEEN

(11) **EP 0 396 734 B1**
(45) Date de publication et mention de la délivrance du brevet: **15.06.1994**
(21) Numéro de dépôt: 90900906.0
(22) Date de dépôt: 20.11.1989
(51) Int. Cl.: A61K 31/445

(54) **COMPOSITIONS PHARMACEUTIQUES POUR LA NEUROPROTECTION CONTENANT DES ARYLCYCLOHEXYLAMINES**
PHARMAZEUTISCHE ZUBEREITUNGEN FÜR NEUROSCHUTZ, DIE ARYLCYCLOHEXYLAMINE ENTHALTEN
PHARMACEUTICAL COMPOSITIONS FOR NEURO PROTECTION CONTAINING ARYLCYCLOHEXYLAMINES

(30) Priorité: 21.11.1988 FR 8815106
(43) Date de publication de la demande: 14.11.1990
(73) Titulaire: CENTRE NATIONAL DE LA RECHERCHE SCIENTIFIQUE (CNRS), 75700 Paris Cedex 07 (FR)
(72) Inventeur: KAMENKA, Jean-Marc, F-34090 Montpellier (FR); PRIVAT, Alain, F-34980 Clément-la-Rivière (FR); CHICHEPORTICHE, Robert, Rubin, F-34070 Montpellier (FR); RONDOUIN, Gérard, F-34000 Montpellier (FR)
(74) Mandataire: Des Termes, Monique
(86) Numéro de dépôt international: FR8900596
(87) Numéro de publication internationale: WO9005524

(56) Documents cités:
- Neurol. Neurobiol., vol. 46, 1988, Alan R. Liss, Inc., J.W. McDonald et al., pp. 601-604
- Neurosci. Lett., vol. 91, no. 2, août 1988, Elsevier Scientific Publishers Ireland Ltd, G. Rondouin et al., pp. 199-203
- Brain Research, vol. 490, 19 juin 1989, Elsevier Science Publishers B.V. (Biomedical Division), J.W. McDonald et al., pp. 33-40
- European J. of Pharmacology, vol. 167, 11 août 1989, Elsevier Science Publishers B.V. (Biomedical Division), M.D. Tricklebank et al., pp. 127-135
- FASEB J., vol. 1, no. 6, 1987, FASEB, M.B. Robinson et al., pp. 446-455
- Brain Research, vol. 152, 1978, J.P. Vincent et al., pp. 176-182
- Pharmacology Biochemistry & Behaviour, vol. 32, pp. 699-705, 1989
- Journal of Neuroscience Research, vol. 29, pp. 133-138, 1991

## Description

La présente invention a pour objet des compositions pharmaceutiques pour la neuroprotection contenant des arylcyclohexylamines.

De façon plus précise, elle concerne l'utilisation d' arylcyclohexylamines ayant une structure proche de celle de la phencyclidine (PCP), c'est-à-dire de la (phényl-1 cyclohexyl)-N pipéridine de formule : 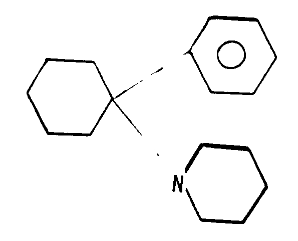
ou de celle de la ((thiényl-2)-1 cyclohexyl)-N pipéridine (TCP) de formule : 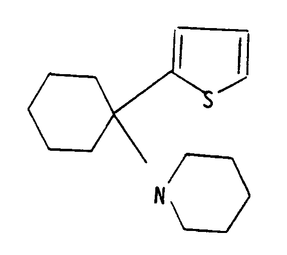
La phencyclidine (PCP) dont l'activité pharmacologique a été étudiée vers 1958, a été introduite comme analgésique et anesthésique sous le nom de Sernyl® et abandonnée ensuite en raison de ses effets secondaires de type psychodysleptiques.

La TCP appartient à ta série des arylcyclohexylamines comme la PCP et elle peut trouver des utilisations en anesthésie comme additif ou pour le traitement de l'hyperexcitabilité en raison de son effet dépressif, comme il est décrit dans le brevet américain US-A-2 921 076 de Parke, Davis & Cy.

Ainsi, ces arylcyclohexylamines n'ont jamais été utilisées en neuroprotection.

Des études récentes portant sur la PCP ont toutefois montré que cette molécule pouvait agir comme antagoniste non compétitif du N-méthyl-D-aspartate (NMDA), ce qui est une propriété intéressante pour la protection cérébrale, notamment contre les dommages associés à la présence de glutamate.

En effet, la toxicité d'acides aminés excitateurs tels que les glutamates, est à la base des principaux dommages du cerveau rencontrés lors d'une ischémie, d'anoxie, d'hypoglycémie ou d'épilepsie. De même, on suppose que la toxicité des aminoacides est impliquée dans certains désordres neurodégénératifs.

Les molécules agissant comme antagonistes du NMDA peuvent trouver une utilisation en neuroprotection.

Cependant, dans le cas de la PCP, les effets secondaires de type psychodysleptique empêchent cette utilisation.

Aussi, des recherches ont été entreprises pour trouver d'autres molécules agissant comme antagoniste du NMDA sans présenter cet inconvénient.

La présente invention a précisément pour objet de nouvelles compositions pharmaceutiques pour la neuroprotection qui contiennent des arylcyclohexylamines ayant un effet antagoniste non compétitif vis-à-vis des récepteurs du NMDA, mais ne présentant pas les effets secondaires gênants de la PCP.

Selon l'invention, la composition pharmaceutique pour la neuroprotection comprend une arylcyclohexylamine répondant aux formules : 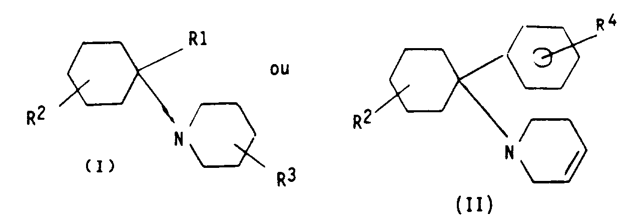
dans lesquelles :
R¹ représente 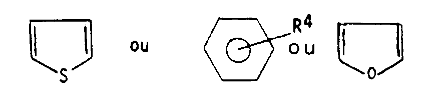
avec R⁴ étant un atome d'hydrogène, un atome de fluor, un atome d'iode ou un radical méthyle,
R² représente un atome d'hydrogène, le radical OH ou le radical CH₃, et
R³ représente un atome d'hydrogène ou le radical CH₂R⁵ avec R⁵ représentant H, OH, Cl, Br ou CH₃COO, à condition que R² et R³ ne soient pas tous deux un atome d'hydrogène, ou un sel d'addition à un acide pharmaceutiquement acceptable de cette arylcyclohexylamine.

Dans cette formule, lorsque R¹ représente le radical thiényle ou furanyle, celui-ci peut être relié au noyau cyclohexyl par un carbone en position ortho ou en position méta par rapport à l'hétéroatome O ou S du radical furanyle ou thiényle.

Les arylcyclohexylamines utilisées dans l'invention ont une structure proche de celle de la PCP ou de la TCP, mais elles s'en différencient par la présence de substituants qui leur confèrent les propriétés de se fixer plus sélectivement sur le site de la phencyclidine en étant des antagonistes non compétitifs du NMDA qui ne se fixent pas sur le site du NMDA, et d'avoir moins d'affinité pour les récepteurs muscariniques et les récepteurs opiacés que la PCP, donc des propriétés psychodysleptiques fortement diminuées.

Ces arylcyclohexylamines sont ainsi des antagonistes non compétitifs du récepteur NMDA des acides aminés excitateurs. Ainsi, elles se fixent sur le récepteur de la PCP et bloquent sélectivement l'entrée des ions Ca²⁺ et Na⁺ qui passent par le canal ionique activé par les agonistes du récepteur NMDA. Cette propriété est à la base des propriétés neuroprotectrices qui peuvent être utilisées pour lutter contre le vieillissement et les atteintes cérébrates aigües. Ainsi, les arylcyclohéxylamines de l'invention peuvent être utilisées dans les cas d'ischémie et de traumatisme cérébral, et également pour la protection contre le vieillissement.

Dans les atteintes cérébrales, on observe une production importante de glutamate qui conduit à la nécrose des cellules. Cette libération est particulièrement importante lorsque la circulation sanguine se rétablit, ce qui engendre une progression de la nécrose des neurones. Cependant, en présence des arylcyclohexylamines de l'invention qui agissent comme antagonistes non compétitifs des récepteurs du NMDA, on observe un blocage des effets toxiques du glutamate.

Certains dérivés de la TCP utilisables dans l'invention ont été décrits par Vincent et al. dans Brain Research, 152, (1978), p.176-182. Cependant dans cet article, on a étudié uniquement l'affinité des dérivés pour les récepteurs muscarinique et opiacé afin de déterminer leurs propriétés psychodysleptiques alors que l'invention est basée en particulier sur l'affet antagoniste non compétitif des composés vis-à-vis des récepteurs du NMDA. Ainsi, on ne pouvait déduire de ce document que les composés de l'invention sont utilisables pour la neuroprotection.

Le brevet GB-A-838 748 (PARKE, DAVIS & Co.) décrit des composés hétérocycliques qui peuvent comprendre également certains dérivés de la TCP utilisables dans l'invention. Cependant, dans ce cas, il s'agit d'additif pour anesthésie et non de médicaments ayant un effet neuroprotecteur.

Ainsi, l'invention a pour objet l'utilisation des arylcyclohexylamines répondant aux formules (I) et (II) données ci-dessus, pour la fabrication de médicaments neuroprotecteurs destinés à empêcher les atteintes cérébrales pouvant se produire lors d'affections aigües telles que l'ischémie, l'anoxie, l'hypoglycémie ou l'épilepsie, ou à protéger le cerveau contre le vieillissement.

Selon un premier mode de réalisation de l'invention, l'arylcyclohexylamine répond à la formule (I) et elle a une structure proche de celle de la PCP. Dans ce cas, R¹ représente un radical de formule : 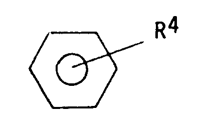
avec R⁴ représentant un atome d'hydrogène, un atome de fluor ou un radical méthyle.

A titre d'exemples de telles arylcyclohexylamines, on peut citer :
- les arylcyclohexylamines de formule (III) : 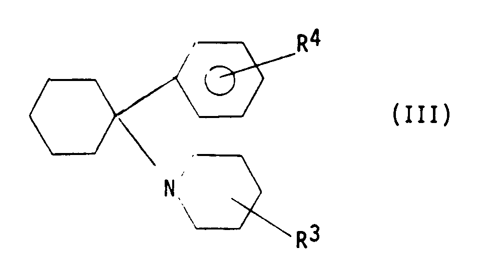 dans laquelle :
   R³ représente te radical méthyle ou le radical CH₂R⁵ avec R⁵ représentant OH ou CH₃COO, et
   R⁴ représente un atome d'hydrogène ;
- les arylcyclohexylamines de formule (IV) 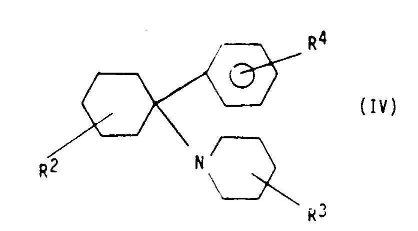 dans laquelle R² est le radical méthyle, R³ est le radical CH₂R⁵ avec R⁵ représentant OH, et R⁴ est un atome de fluor ; et
- les arylcyclohexylamines de formule : 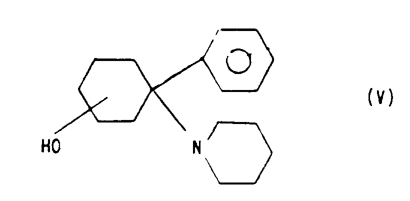
Selon un second mode de réalisation de l'invention, l'arylcyclohexylamine répond à la formule (I) et a une structure proche de celle de la TCP. Dans ce cas, R¹ représente le radical thiényle qui peut être relié au noyau cyclohexyle par l'intermédiaire d'un carbone en position ortho ou en position méta par rapport à l'atome de S du radical thiényle.

A titre d'exemples de telles arylcyclohexylamines, on peut citer :
- les arylcyclohexylamines de formule (VI) 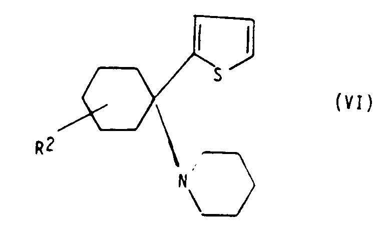 dans laquelle R² est le radical méthyle,
- les arylcyclohexylamines de formule (VII) 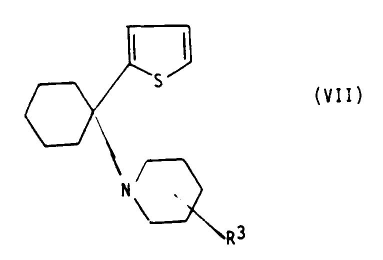 dans laquelle R³ est le radical CH₂R⁵ avec R⁵ représentant OH ou Cl,
- les arycyclohexylamines de formule (VIII) 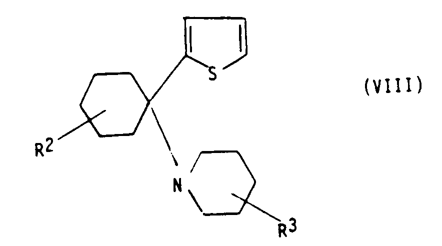 dans laquelle R² est le radical méthyle et R³ est le radical CH₂OH.

Dans les arylcyclohexylamines de l'invention, la position des substituants R², R³ et R⁴ a un effet sur les propriétés obtenues.

De préférence, lorsque R² est le radical CH₃, il est en position ortho. Lorsque R² est le radical OH, il est de préférence également en position ortho.

Lorsque R³ est le substituant CH₂R⁵, sa position par rapport à l'atome d'azote a également un effet sur le résultat obtenu. Généralement, il est en position méta ou para, de préférence en position méta.

Lorsque R¹ est un radical phényle substitué, le substituant R⁴ est de préférence en position méta.

Les arylcyclohexylamines utilisées dans l'invention peuvent être sous la forme de différents stéréoisomères, et tous ces stéréoisomères conviennent dans l'invention. Toutefois, lorsque R² n'est pas un atome d'hydrogène, et se trouve en position ortho ou méta, on préfère généralement utiliser les isomères cis/pipéridine qui sont les plus efficaces. Lorsque R² n'est pas un atome d'hydrogène et se trouve en para, on préfère utiliser les isomères trans/pipéridine.

Ainsi, on utilise de préférence l'arylcyclohexyl aminé de formule : 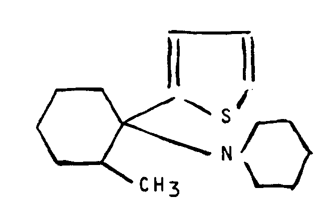
avec CH₃ en position cis par rapport à la pipéridine.

Certaines arylcyclohexylamines de l'invention peuvent également exister sous forme d'isomères optiques, et l'on peut utiliser dans l'invention soit les isomères optiques seuls, soit le mélange racémique .

L'invention a encore pour objet un procédé pour empêcher les atteintes cérébrales chez l'homme ou chez l'animal, qui consiste à administrer à l'homme ou à l'animal une quantité thérapeutiquement efficace d'une arylcyclohexylamine répondant aux formules I ou II données ci-dessus, ou de TCP dans le cas de lutte contre le vieillissement cérébral.

Les arylcyclohexylamines de l'invention peuvent être préparées par des procédés classiques, par exemple à partir des composés suivants :
- les pipéridines de formule : 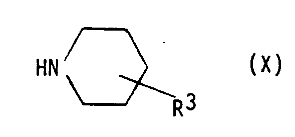 dans laquelle R³ a la signification donnée ci-dessus,
- la tétrahydropyridine de formule : 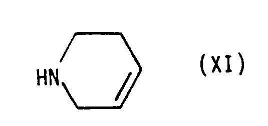
- les cyclohexanones de formule : 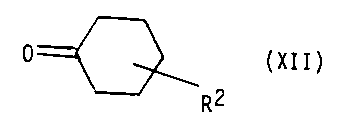 dans laquelle R² a la signification donnée ci-dessus,
- les halogénures de benzène de formule : 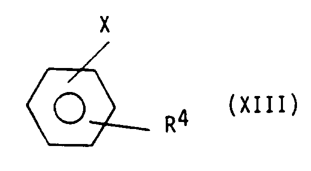 dans laquelle X représente un atome d'iode, de brome ou de chlore et R⁴ a la signification donnée ci-dessus, et
- les halogénures de thiophène de formule : 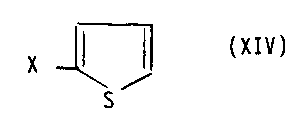 dans laquelle X a la signification donnée ci-dessus.

Plusieurs procédés de synthèse peuvent être envisagés et leur choix dépend en particulier de la position du substituant R² et de l'isomère que l'on veut obtenir.

On décrit, ci-après, trois voies de synthèse utilisables.

### Voie de synthèse I.

Cette voie de synthèse se déroule en deux étapes qui sont respectivement :
a) la préparation d'alpha-aminonitriles à partir des pipéridines de formule (X) ou pyridines de formule (XI) et des cyclohexanones de formule (XII), puis
b) la réaction des alpha-aminonitriles avec les halogénures de benzène de formule (XIII) ou les halogénures de thiophène de formule (XIV).

L'alpha -aminonitrile répond à la formule : 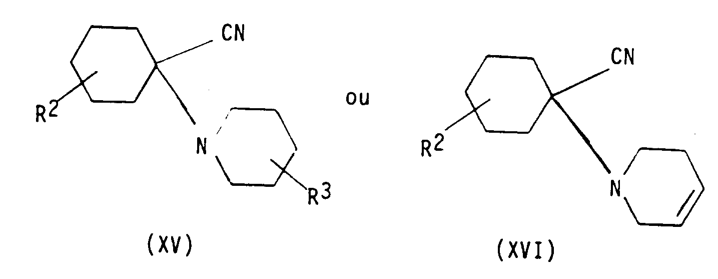
dans lesquelles R² et R³ ont la signification donnée ci-dessus.

Il est préparé par une variante de la réaction de Strecker qui se déroule en milieu organique anhydre.

Dans ce but, on fait réagir de l'acétone cyanhydrine (donneur d'ions cyanure) avec 1°) la pipéridine de formule (X) ou la pyridine de formule (XI), et 2°) une cyclohexanone de formule (XII), en présence de sulfate de magnésium qui agit comme déshydratant, dans un solvant du type diméthylacétamide (DMA). Après traitement et purification, on isole l'alpha-aminonitrile correspondant avec une pureté d'au moins 95% et on l'utilise tel quel dans la suite de la synthèse.

Dans la deuxième étape, on fait réagir cet alpha-aminonitrile avec un halogénure de benzène de formule (XIII) ou un halogénure de thiophène de formule (XIV) en utilisant la synthèse dite de Bruylants.

Cette synthèse consiste à faire réagir l'alpha-aminonitrile avec un réactif de Grignard obtenu à partir de l'halogénure de benzène ou de thiophène de formule (XIII) ou (XIV) dans l'éther ou le tétrahydrofurane (THF) anhydre. Après traitement convenable et purification, on isole l'arylcyclohexylamine recherchée. On fait ensuite barboter du gaz chlorhydrique anhydre dans une solution éthérée de l'arylcyclohexylamine pure, et on précipite ainsi le chlorhydrate hydrosoluble correspondant.

Cette voie de synthèse est stéréospécifique ; elle ne permet donc d'accéder qu'à un seul des isomères possibles.

Ainsi, lorsque le substituant R² est en ortho ou para, on obtient l'isomère cis / pipéridine ; lorsque R² est en position méta, on obtient l'isomère trans.

### Voie de synthèse II.

Cette synthèse se déroule en 4 étapes qui sont les suivantes :
a) préparation d'alcool benzylique de formule : 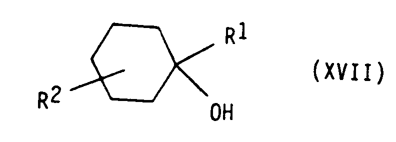 On fait réagir une cyclohexanone de formule XII avec un réactif de Grignard obtenu à partir des halogénures de benzène ou de thiophène de formule XIII ou XIV dans de l'éther anhydre. On obtient ainsi les alcools benzyliques que l'on soumet à une rapide purification.
b) Préparation des dérivés azides correspondants de formule : 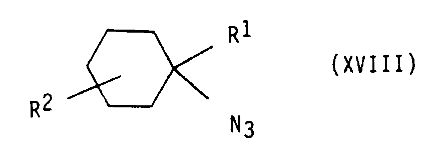 Selon un processus proche de celui utilisé dans les réactions de Schmidt et de Curtius, on effectue la substitution de la fonction alcool par la fonction azide en ajoutant les alcools bruts précédents à une suspension d'azidure de sodium dans l'acide trichloracétique et le chloroforme à froid. Après traitement, on isole un mélange brut d'azides épimères que l'on utilise tel quel dans la suite.
c) réduction des azides.
   On fait réagir le mélange précédent avec du nickel de Raney dans de l'isopropanol pour fournir après traitement un mélange d'amines primaires épimères utilisé tel quel dans la suite.
d) Formation du cycle pipéridinique et purification.
   On fait réagir dans l'acétonitrile à chaud le dibromo-1,5 pentane sur le mélange précédent des amines primaires. Après traitement, on isole un résidu contenant essentiellement un mélange brut de deux stéréoisomères. On peut séparer les deux isomères purs par chromatographie et obtenir ainsi le composé recherché. Par barbotage de gaz chlorhydrique anhydre dans la solution éthérée du dérivé séparé, on précipite le chlorhydrate hydrosoluble correspondant.

Ainsi, la voie II permet d'accéder aux deux isomères possibles et tout particulièrement à celui non obtenu par la voie I.

Cependant la voie II ne peut être utilisée pour la préparation des dérivés de formule (II)ou pour la préparation des dérivés de formule (I)comportant un substituant R³.

### Voie de synthèse III.

Cette voie de synthèse comprend les étapes suivantes :
a) formation d'alcool benzylique de formule XVII comme dans la voie de synthèse II par réaction d'une cyclohexanone de formule XII dans laquelle R² est un atome d'hydrogène, avec le réactif de Grignard obtenu à partir de l'halogénure de benzène ou de thiophène de formule XIII ou XIV. On purifie rapidement l'alcool et on l'utilise tel quel dans la suite de la synthèse.
b) Formation d'arylcyclohexène de formule : 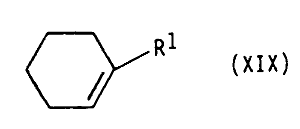 On déshydrate en milieu acide l'alcool obtenu précédemment pour fournir l'arylcyclohexène recherché.
c) Formation des époxydes correspondants.
   On forme l'époxyde correspondant de formule : 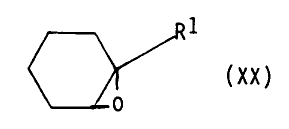 en utilisant un peracide approprié
d) Ouverture des époxydes par la pipéridine.
   L'époxyde précédemment obtenu est ouvert à chaud par la pipéridine, en milieu méthanolique, pour donner un alcool benzylique majoritaire et une hydroxy-2 arylcyclohexylamine minoritaire de formule : 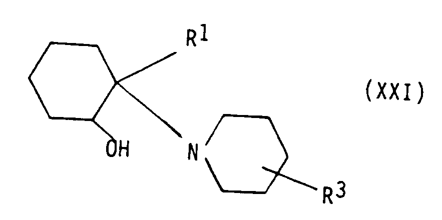 constituant le produit recherché.

Par barbotage de gaz chlorhydrique anhydre dans la solution éthérée du produit obtenu, on précipite le chlorhydrate hydrosoluble correspondant.

Cette voie III est elle aussi stéréospécifique et conduit à l'isomère avec OH trans/pipéridine ; de plus, elle ne permet d'obtenir commodément que des composés hydroxylés en position 2 qui ne peuvent se préparer par les deux autres voies.

Pour préparer le médicament de l'invention, on peut utiliser les arylcyclohexylamines répondant aux formules (I) ou (II) ci-dessus, ou leurs sels d'addition aux acides pharmaceutiquement acceptables.

Les acides utilisables sont par exemple l'acide chlorhydrique, l'acide sulfurique et l'acide tartrique.

Généralement, on mélange l'arylcyclohexylamine ou l'un de ses sels d'addition aux acides pharmaceutiquement acceptables avec un véhicule ou support approprié, solide ou liquide, pharmaceutiquement acceptable, et éventuellement avec d'autres additifs pharmaceutiquement acceptables.

Ainsi, pour la préparation de ces médicaments, on peut dissoudre un sel d'addition aux acides de l'arylcyclohexylamine dans une solution aqueuse injectable ou administrable par voie orale, par exemple du sérum physiologique.

On peut aussi inclure les arylcyclohexylamines de l'invention dans des préparations solides pour obtenir par exemple des comprimés, administrables par voie orale, ou des médicaments-retard sous forme d'implant, en utilisant des techniques classiques.

Les doses généralement administrées peuvent varier de 0,1 à 5mg/kg. Lorsque la composition pharmaceutique est sous la forme d'une solution, la concentration de la solution en arylcyclohexylamine est telle que le volume administré varie de 0,5 à 2ml.

Lorsque le médicament de l'invention est utilisé pour le traitement d'affections cérébrales aigües par exemple dans les cas d'ischémie, d'hypoglycémie ou d'épilepsie, on préfère généralement administrer une dose relativement élevée en une seule fois, par exemple par injection intraveineuse, pour obtenir l'effet maximum.

Dans le cas où on utilise le médicament pour la protection contre le vieillissement, les doses sont généralement plus faibles et peuvent être administrées une à deux fois par mois.

Pour cette utilisation, ou préfèrera les modes d'administration utilisant des comprimés ou des implants.

D'autres caractéristiques et avantages de l'invention apparaitront mieux à la lecture des exemples suivants donnés bien entendu à titre illustratif en référence au dessin annexé sur lequel
- la figure 1 est un diagramme représentant le taux de survie des neurones dans une culture "in vitro" traitée par différents composés, et
- les figures 2 à 5 sont des représentations micrographiques des coupes de cerveaux de souris traitées et non traitées.

### Exemple 1 : Préparation de cyano-1((méthyl-4 pipéridino)-1)-1 cyclohexane (synthon I).

On mélange 9,8g (0,1M) de cyclohexanone, 13,4g (0,15M) d'acétone cyanhydrine, 63g (0,5M) de MgSO₄ (séché) dans 14,9g (0,15M) de méthyl-4 pipéridine et 8,7g (0,1M) de DMA (diméthylacétamide). On agite fortement à 45° pendant 48h, puis on jette dans un grand volume d'eau et de glace violemment agité pendant 30min. Après extraction à l'éther, séchage (Na₂CO₃), évaporation du solvant sous vide, on obtient un résidu solide qui après une cristallisation dans l'hexane ou l'éther de pétrole donne 15,5g (75%) du synthon I sous la forme d'un solide blanchâtre de pureté égale ou supérieure à 95%, suffisante pour la suite des synthèses, qui est identifié par spectrométrie infrarouge.

### Exemple 2 : Préparation du cyano-1 ((tétrahydro-1,2,5,6 pyridino)-1)-1 cyclohexane (synthon II).

On mélange 15,1g (0,15M) de cyclohexanone, 13,9g (0,16M) d'acétone cyanhydrine, 92g (0,77M)de MgSO₄ (séché) dans 22g (0,3M) de tétrahydro-1,2,5,6 pyridine et 26,1g (0,3M) de DMA. On agite fortement à 45° pendant 48h, puis on jette dans un grand volume d'eau et de glace violemment agité pendant 30min. Après extraction à l'éther, séchage (Na₂CO₃), évaporation du solvant sous vide, on obtient un résidu solide brun-jaune qui après une cristallisation dans l'hexane ou l'éther de pétrole donne 20g (70%) du synthon II sous la forme d'un solide jaunâtre de pureté au moins égale à 95%, suffisante pour la suite des synthèses, qui est identifié par spectrométrie infrarouge et spectrométrie de masse couplée à la chromatographie gazeuse (GC/MS).

Pour la GC/MS, on utilise une colonne capillaire OVI de 25m. Les conditions et les résultats obtenus sont les suivants :
Temp. Injecteur de 100 à 250°C (20°C/min),
Temp. Four : 200°C,
Temps de rétention : 4,84min, m/e 190,2.

### Exemple 3 : Préparation du cyano-1 (hydroxyméthyl-3 pipéridino)-1 cyclohexane (synthon III).

On mélange 20g (0,2M) de cyclohexanone, 26g (0,3M) d'acétone cyanhydrine, 49g (0,4M) de MgSO₄ (séché) dans 30,3g (0,3M) d'hydroxyméthyl-3 pipéridine et 17,8g (0,2M) de DMA. On agite fortement à 45° pendant 48h puis on jette dans un grand volume d'eau et de glace violemment agité pendant 30min. On obtient un précipité blanc que l'on essore et sèche sous vide. On isote ainsi 23,1g (85%) d'aminonitrile (synthon III) de pureté ≧ 95%, suffisante pour la suite des synthèses, qui est identifié par spectrométrie infrarouge, et spectrométrie de masse couplée à la chromatographie gazeuse (GC/MS).

Pour la GC/MS, on utilise une colonne capillaire OV1 de 25m. Les conditions et les résultats obtenus sont les suivants :
Temp. Injecteur de 50 à 230°C (10°C/min),
Temp. Four : 250°C
Temps de rétention : 13,92min, m/e 222,1.

### Exemple 4 : Préparation du cyano-1 r-(hydroxyméthyl-3 pipéridino)-1)-1-t-méthyl-4 cyclohexane (synthon IV).

On mélange 15g (0,13M) de méthyl-4 cyclohexanone, 11,3g (0,13M) d'acétone cyanhydrine, 80,4g (0,67M)de MgSO₄ (séché) dans 30,8g (0,27M) d'hydroxyméthyl-3 pipéridine et 11,3g (0,13M) de DMA. On agite fortement à 45° pendant 48h, puis on jette dans un grand volume d'eau et de glace violemment agité pendant 30min. Après extraction à l'éther, séchage (Na₂CO₃), évaporation du solvant sous vide, on obtient un résidu solide blanchâtre qui après une cristallisation dans l'hexane ou l'éther de pétrole donne 22,2g (70%) d'un solide blanc de pureté ≧ 95% suffisante pour la suite des synthèses. Ce composé contient le synthon IV et une faible proportion du second stéréoisomère possible, ce qui ne présente pas d'inconvénient dans la suite puisqu'en présence d'un réactif de Grignard (acide de Lewis), il s'épimérise en isomère majoritaire qui est seul à réagir.

### Exemple 5 : Préparation du ((méthyl-4 pipéridino)-1)-1 phényl-1 cyclohexane de formule

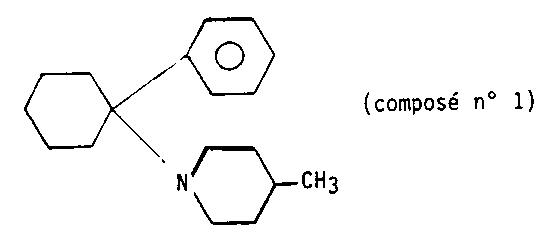

On prépare, dans 50ml d'éther anhydre, le réactif de Grignard résultant de l'action de 8g (0,05M) de bromobenzène sur 2g (0,08M) de magnésium en tournures. On y ajoute, lentement, 5g (0,02M) de synthon I dissous dans 50ml d'éther anhydre. On agite 12h au reflux, décompose le complexe par une solution saturée froide de NH₄Cl, puis, après décantation, on extrait les eaux à l'éther (3x50ml). Les phases éthérées réunies sont extraites par une solution aqueuse de HCl 20% (2x50ml). Les eaux acides sont neutralisées par NH₄OH, extraites à l'éther (3x50ml) ; les éthers rassemblés, après séchage (Na₂SO₄), sont évaporés sous vide pour donner un résidu solide jaunâtre de 5,8g. Ce dernier est chromatographié sur alumine, dans l'éther de pétrole pur, pour donner 4,8g (77%) de composé n^{o} 1 sous la forme d'un solide blanc, analytiquement pur, fondant à 53-54°. Par barbotage de HCl gazeux dans la solution éthérée du composé n^{o} 1, on fait précipiter son chlorhydrate, solide blanc qui, récupéré par essorage et séché sous vide, fond à 211-212°.

Analyse centésimale du produit de formule brute C₁₈H₂₈NCl :

| | trouvé | calculé |
|---|---|---|
| C | 73,25 | 73,60 |
| H | 9,78 | 9,54 |
| N | 4,96 | 4,77 |

### Exemple 6 : Préparation du ((tétrahydro-1,2,5,6 pyridino)-1)-1 (thiényl-2)-1 cyclohexane de formule :

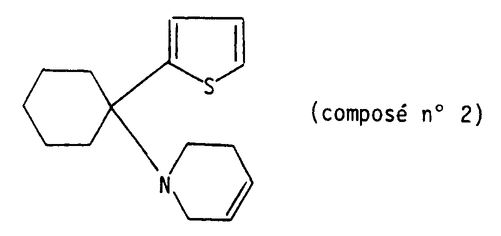

On prépare, dans 50ml d'éther anhydre, le réactif de Grignard résultant de l'action de 8,15g (0,05M) de bromo-2 thiophène sur 2g (0,08M) de magnésium en tournures. On y ajoute, lentement, 2g (0,01M) de synthon II dissous dans 50ml d'éther anhydre. On agite 12h au reflux, décompose te complexe par une solution saturée froide de NH₄Cl, puis, après décantation, on extrait tes eaux à l'éther (3x50ml). Les phases éthérées réunies sont extraites par une solution aqueuse de HCl 20% (2x50ml). Les eaux acides sont neutralisées par NH₄OH, extraites à l'éther (3x50ml) ; les éthers rassemblés, après séchage (Na₂SO₄), sont évaporés sous vide pour donner un résidu solide jaune-brun de 2g. Ce dernier est chromatographié sur silice dans l'éther de pétrole pur, pour donner 1,3g (53%) de composé n^{o} 2 sous la forme d'un solide blanc, analytiquement pur, fondant à 46-48°. Par barbotage de HCl gazeux dans la solution éthérée de ce composé, on fait précipiter son chlorhydrate, solide blanc qui, récupéré par essorage et séché sous vide, fond à 163-164°C.

Analyse centésimale du produit de formule brute C₁₅H₂₂NSCL :

| | trouvée | calculée |
|---|---|---|
| C | 63,62 | 63,49 |
| H | 7,97 | 7,76 |
| N | 4,68 | 4,94 |

### Exemple 7 : Préparation de l'[(hydroxyméthyl-3 pipéridino)-1]-1 (thiényl-2)-1 cyclohexane de formule :

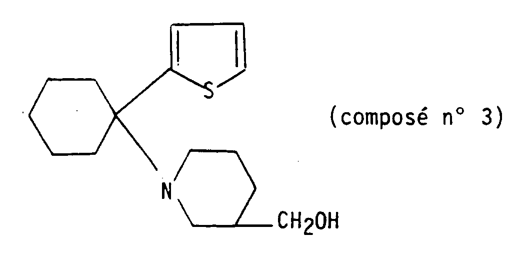

On prépare, dans 350ml d'éther anhydre, le réactif de Grignard résultant de l'action de 57,6g (0,35M) de bromo-2 thiophène sur 9g (0,38M) de magnésium en tournures. On y ajoute, lentement, 15,7g (0,07M) de synthon III dissous dans 350ml d'éther anhydre. On agite 12h au reflux, décompose le complexe par une solution saturée froide de NH₄Cl, puis, après décantation, on extrait à l'éther (3x250ml). Les phases éthérées réunies sont extraites par une solution aqueuse de HCl 20% (2x200ml). Les eaux acides sont neutralisées par NH₄OH, extraites à l'éther (3x250ml) ; les éthers rassemblés, après séchage (Na₂SO₄), sont évaporés sous vide pour donner un résidu huileux jaunâtre de 16,3g. Ce dernier est chromatographié sur chromatographe préparatif haute performance (2 opérations) sur silice, dans l'éther de pétrole contenant de l'éther (80/20 v/v), pour donner 14,5g (74%) du composé n^{o} 3 sous la forme d'huile claire, analytiquement pure. Par barbotage de HCl gazeux dans la solution éthérée de ce composé, on fait précipiter son chlorhydrate, solide blanc qui, récupéré par essorage et séché sous vide, fond à 170-171° (analytiquement pur).

Analyse centésimale du produit de formule brute C₁₆H₂₆NOSCl :

| | trouvée | calculée |
|---|---|---|
| C | 60,59 | 60,86 |
| H | 8,11 | 8,24 |
| N | 4,16 | 4,44 |

### Exemple 8 : Préparation de l'[(hydroxyméthyl-3 pipéridino)-1]-1 phényl-1 cyclohexane de formule :

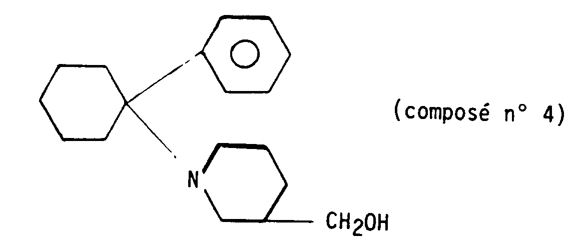

On prépare, dans 350ml d'éther anhydre, le réactif de Grignard résultant de l'action de 55,5g (0,35M) de bromobenzène sur 9g (0,38M) de magnésium en tournures. On y ajoute, lentement, 15,7g (0,07M) de synthon III dissous dans 350ml d'éther anhydre. On agite 12h au reflux, décompose le complexe par une solution saturée froide de NH₄Cl, puis, après décantation, on extrait à l'éther (3x250ml). Les phases éthérées réunies sont extraites par une solution aqueuse de HCl 20% (2x200ml). Les eaux acides sont neutralisées par NH₄OH, extraites à l'éther (3x250ml) ; les éthers rassemblés, après séchage (Na₂SO₄), sont évaporés sous vide pour donner un résidu huileux blanchâtre de 15,9g. Ce dernier est chromatographié sur chromatographe préparatif haute performance (2 opérations), sur silice, dans l'éther de pétrole contenant de l'éther (80/20 v/v), pour donner 13,5g (70%) du composé n^{o} 4 sous la forme d'huile claire, analytiquement pure. Par barbotage de HCl gazeux dans la solution éthérée de ce composé, on fait précipiter son chlorhydrate, solide blanc qui, récupéré par essorage et séché sous vide, fond à 205-207° (analytiquement pur).

Analyse centésimale du produit de formule brute C₁₈H₂₈NOCl :

| | trouvée | calculée |
|---|---|---|
| C | 70,02 | 69,79 |
| H | 9,24 | 9,05 |
| N | 4,55 | 4,52 |

### Exemple 9 : Préparation du r-(hydroxyméthyl-3 pipéridino)-1-c-méthyl-4 (thiényl-2)-1 cyclohexane de formule :

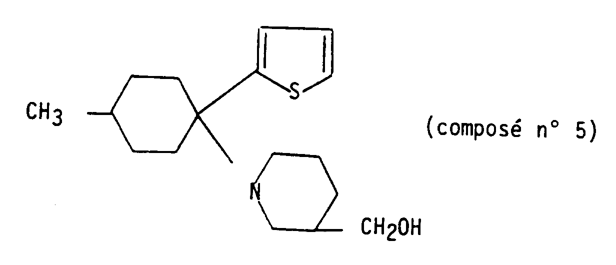

On prépare dans 200ml d'éther anhydre, le réactif de Grignard résultant de l'action de 31g (0,19M) de bromo-2 thiophène sur 4,8g (0,20M) de magnésium en tournures. On y ajoute lentement 9g (0,04M) de synthon IV dissous dans 200ml d'éther anhydre. On agite 12h au reflux, décompose le complexe par une solution saturée froide de NH₄Cl, puis, après décantation, on extrait les eaux à l'éther (3x150ml). Les phases éthérées réunies sont extraites par une solution aqueuse de HCl 20% (2x150ml). Les eaux acides sont neutralisées par NH₄OH, extraites à l'éther (3x150ml) ; les éthers rassemblés, après séchage (Na₂SO₄), sont évaporés sous vide pour donner un résidu huileux jaunâtre de 9g. Ce dernier est chromatographié sur chromatographe préparatif haute performance sur silice, dans l'hexane contenant de l'éther (95/5 v/v), pour donner 7,5g (67%) du composé n^{o}5 sous la forme d'huile claire analytiquement pure. Par barbotage de HCl gazeux dans la solution éthérée de ce composé, on fait précipiter son chlorhydrate, solide blanc qui, récupéré par essorage et séché sous vide, fond à 186-187° (analytiquement pur).

Analyse centésimale du produit de formule brute C₁₇H₂₉NOSCl :

| | trouvée | calculée |
|---|---|---|
| C | 61,62 | 61,91 |
| H | 8,56 | 8,50 |
| N | 4,34 | 4,25 |

### Exemple 10 : Préparation de l'acétate de ((hydroxyméthyl-3-pipéridino)-1)-1 phényl-1 cyclohexane de formule :

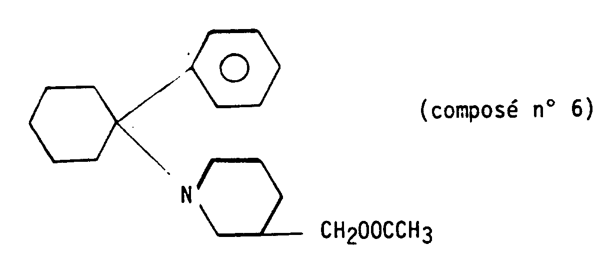

3g (0,01M) du composé n^{o} 4 sont placés dans un mélange de 3g (0,03M) d'anhydride acétique et 2g (0,03M) de pyridine anhydre. La solution est agitée à 25° pendant 24h, puis jetée dans un grand volume d'eau et de glace que l'on agite violemment pendant 30min. La solution est extraite à l'éther (3x50ml), les éthers séchés (Na₂SO₄) sont évaporés sous vide pour laisser un résidu huileux blanchâtre de 3,3g. Ce dernier est chromatographié sur alumine dans l'éther de pétrole pur pour donner 2,7g (78%) de composé n^{o} 6 sous la forme d'une huile claire, analytiquement pure. Par barbotage de HCl gazeux dans la solution éthérée de ce composé, on fait précipiter son chlorhydrate, solide blanc qui, récupéré par essorage et séché sous vide, se décompose à 180° (analytiquement pur).

Analyse centésimale du produit de formule brute C₂₀H₃₀NO₂Cl :

| | trouvée | calculée |
|---|---|---|
| C | 68,25 | 68,28 |
| H | 8,84 | 8,54 |
| N | 3,66 | 3,98 |

### Exemple 11 : Préparation du ((fluoro-3 phényl)-1)-1-r-((hydroxyméthyl-3 pipéridino)-1)-1-c-méthyl-4 cyclohexane de formule :

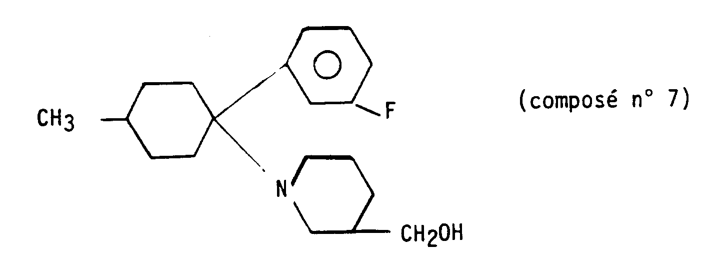

On prépare, dans 50ml d'éther anhydre, le réactif de Grignard résultant de l'action de 7,5g (0,04M) de bromo-1 fluoro-3 benzène sur 1g (0,04M) de magnésium en tournures. On y ajoute, lentement, 2g (0,08M) de synthon IV dissous dans 50ml d'éther anhydre. On agite 12h au reflux et décompose le complexe par une solution saturée froide de NH₄Cl, puis, après décantation, on extrait les eaux à l'éther (3x50ml). Les phases éthérées réunies sont extraites par une solution aqueuse de HCl 20% (2x50ml). Les eaux acides sont neutralisées par NH₄OH, extraites à l'éther (3x50ml) ; les éthers rassemblés, après séchage (Na₂SO₄), sont évaporés sous vide pour donner un résidu huileux brunâtre de 1,7g. Ce dernier est chromatographié sur chromatographe préparatif haute performance sur silice, dans le pentane contenant de l'éther (80/20 v/v), pour donner 1,2g (46%) du composé n^{o} 7 sous la forme d'huile claire, analytiquement pure. Par barbotage de HCl gazeux dans la solution éthérée de ce composé, on fait précipiter son chlorhydrate, solide blanc qui, récupéré par essorage et séché sous vide, se décompose à 218° (analytiquement pur).

Analyse centésimale du produit de formule brute C₁₉H₂₉NOFCl :

| | trouvée | calculée |
|---|---|---|
| C | 66,68 | 66,76 |
| H | 8,39 | 8,49 |
| N | 3,88 | 4,10 |

### Exemple 12 : Préparation du [(chlorométhyl-3 pipéridino)-1]-1 (thiényl-2)-1 cyclohexane de formule :

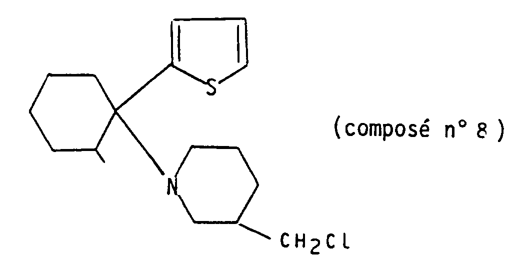

A 2,5g (0,009M) du composé n^{o} 3 et 1ml de chlorure de méthylène, on ajoute très lentement (avec évacuation ou piégeage pour gaz acides), sous agitation, 1g de chlorure de thionyle dissous dans 1ml de chlorure de méthylène. On chauffe ensuite 6h à 60°, puis évapore le solvant sous vide. Le résidu obtenu est dilué dans 10ml d'eau et neutralisé avec précaution par NH₄OH 20%. On extrait tes eaux à l'éther (3x20ml), sèche les éthers rassemblés (Na₂SO₄) et les évapore sous vide ce qui fournit un résidu huileux brunâtre de 2,4g. Celui-ci est purifié par flash chromatographie sur silice, dans l'éther de pétrole, pour donner 2,1g (78%) du composé n^{o} 8 sous la forme d'huile claire analytiquement pure. Par barbotage de HCl gazeux dans la solution éthérée de ce composé, on fait précipiter son chlorhydrate, solide blanc qui, récupéré par essorage et séché sous vide, fond à 150-151° (analytiquement pur).

Analyse centésimale du produit de formule brute C₁₆H₂₅NSCl₂ :

| | trouvée | calculée |
|---|---|---|
| C | 57,18 | 57,49 |
| H | 7,58 | 7,49 |
| N | 4,28 | 4,19 |

### Exemple 13 : Préparation du c-méthyl-2-r-(pipéridino-1)-1 (thiényl-2)-1 cyclohexane de formule :

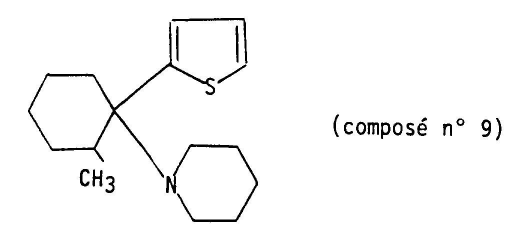

A) On prépare, dans 75ml d'éther anhydre, le réactif de Grignard résultant de l'action de 8,25g (0,05M) de bromo-2 thiophène sur 2g (0,08M) de magnésium en tournures. On y ajoute 5,6g (0,05M) de méthyl-2 cyclohexanone dissous dans 75ml d'éther anhydre. On agite 3h au reflux, décompose le complexe par une solution saturée froide de NH₄Cl, puis, après décantation, on extrait les eaux à l'éther (3x50ml). Les éthers, après séchage (Na₂SO₄), sont évaporés sous vide pour donner 8,7g (88,8%) de mélange d'alcools épimères (Ir). La réaction suivante procédant par carbocation, les alcools ne sont pas autrement purifiés.
B) On prépare à -5° une suspension contenant 6,5g (0,1M) d'azidure de sodium, 82g (0,5M) d'acide trichloracétique et 100ml de chloroforme fortement agitée. On y ajoute, lentement, dissous dans 70ml de chloroforme et à la même température, 8,7g (0,045M) des alcools obtenus précédemment L'agitation et la température sont maintenues pendant 3h (ou jusqu'à disparition des alcools), puis on neutralise, à froid, par NH₄OH, décante, extrait la phase aqueuse ou chloroforme (2x50ml) et lave les phases organiques rassemblées jusqu'à pH neutre. Après séchage (Na₂SO₄), évaporation sous vide, on récupère un résidu huileux pesant 8,85g, contenant essentiellement deux dérivés insaturés (très minoritaires) et deux azides épimères (IR) qui, compte tenu de leur relative instabilité, ne sont pas autrement purifiés.
C) Le mélange des deux azides précédemment obtenu de 8,85g est dissous dans 100ml d'isopropanol et chauffé à 65°. On ajoute par portions du Nickel de Raney (en maintenant la température) jusqu'à cessation de dégagement gazeux. On chauffe alors à 70° pendant 15min, refroidit à la température ambiante, filtre sur célite, lave celle-ci avec 100ml d'isopropanol, ajoute aux phases alcooliques 200ml d'HCl à 20% et évapore sous vide pour éliminer l'isopropanol. La phase aqueuse restante, refroidie, est neutralisée par NH₄OH. On extrait ensuite à l'éther (3x50ml), sèche (Na₂CO₃) et évapore le solvant sous vide pour obtenir un résidu huileux de 4,6g qui contient essentiellement deux amines primaires épimères (IR, GC/MS).
D) Les amines précédentes, 4,6g, sont dissoutes dans 100ml d'acétonitrile contenant 5,2g de dibromo-1,5 pentane et 13g de K₂CO₃. Le mélange, fortement agité, est porté au reflux pendant 72h puis refroidi à température ambiante. Après filtration, on ajoute 150ml d'HCl 20%, extrait à l'éther (2x50ml), les eaux acides, neutralisées par NH₄OH, sont à leur tour extraites à l'éther (3x50ml). Après séchage (Na₂CO₃), les éthers sont évaporés sous vide pour donner un résidu huileux de 3,7g contenant essentiellement deux amines tertiaires isomères (IR, GC/MS). Le mélange obtenu est chromatographié sur chromatographe préparatif haute performance sur silice, dans l'hexane contenant de l'éther (95/5 v/v) pour donner une première fraction d'huile claire qui cristallise lentement (40-41°), analytiquement pure, de 1,7g de t-méthyl-2-r-(pipéridino-1)-1 (thiényl-2)-1 cyclohexane ; une deuxième fraction de cristaux blancs de 1,4g (80-81°), analytiquement pure, du composé n^{o} 9 ou c-méthyl-2-r-(pipéridino-1)-1 (thiényl-2)-1 cyclohexane et deux autres fractions de 400mg au total, constituées d'amines primaires n'ayant pas réagi dont le mélange est recyclable lors d'une synthèse ultérieure. Par barbotage de HCl gazeux dans la solution éthérée des bases cristallisées, on fait précipiter leurs chlorhydrates, solides blancs qui, récupérés par essorage et séchés sous vide, fondent respectivement à 152-153° et 220-221° pour le composé n^{o} 9, HCl. Le rendement en composé n^{o} 9 base à partir de la cétone de départ est de 10,5%. Les deux structures stéréoisomères sont aisément différenciées par RMN du ¹³C (CDCl₃) des chlorhydrates (conformations anancomères) : dans le composé n^{o} 9, HCl, le méthyle, les carbones C2, C4 et C6 sont plus blindés que dans l'isomère de plus bas point de fusion, en raison de la position axiale du méthyle (au lieu d'équatoriale dans l'autre isomère).
Analyse centésimale du produit de formule brute C₁₆H₂₆NSCl :

| | trouvée | calculée |
|---|---|---|
| C | 64,23 | 64,11 |
| H | 8,69 | 8,68 |
| N | 4,59 | 4,67 |

### Exemple 14 : Préparation du t-méthyl-4-r-(pipéridino-1)-1 (thiényl-2)-1 cyclohexane de formule :

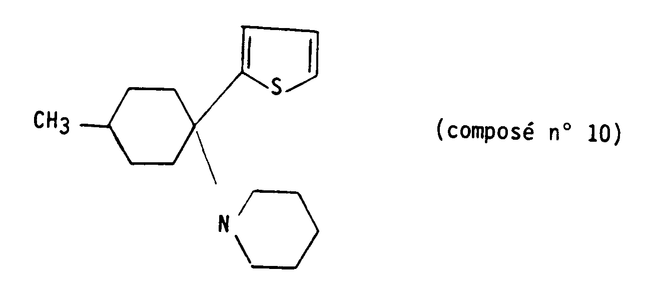

A) On prépare, dans 300ml d'éther anhydre, le réactif de Grignard résultant de l'action de 49,5g (0,3M) de bromo-2 thiophène sur 8,75g (0,35M) de magnésium en tournures. On y ajoute 22,4g (0,2M) de méthyl-4 cyclohexanone dissous dans 300ml d'éther anhydre. On agite 3h au reflux, décompose le complexe par une solution saturée froide de NH₄Cl, puis, après décantation, on extrait les eaux à l'éther (3x200ml). Les éthers, séchés (Na₂SO₄), sont évaporés sous vide pour donner un solide jaunâtre qui, après une cristallisation dans l'éther de pétrole, donne un solide blanc de 31,4g (80%) de mélange d'alcools épimères (IR). La réaction suivante procédant par carbocation, les alcools ne sont pas autrement purifiés.
B) On prépare à -5° une suspension contenant 4,9g (0,075M) d'azidure de sodium, 61g (0,37M) d'acide trichloracétique et 35ml de chloroforme et fortement agitée. On y ajoute, lentement, dissous dans 50ml de chloroforme et à la même température, 6g (0,031M) des alcools obtenus précédemment. L'agitation et la température sont maintenues pendant 3h (ou jusqu'à disparition des alcools), puis on neutralise, à froid, par NH₄OH, décante, extrait la phase aqueuse au chloroforme (2x50ml) et lave les phases organiques rassemblées jusqu'à pH neutre. Après séchage (Na₂SO₄), évaporation sous vide, on récupère un résidu huileux pesant 5,75g, contenant essentiellement un dérivé insaturé (très minoritaire) et deux azides épimères (Ir) qui, compte tenu de leur relative instabilité, ne sont pas autrement purifiés.
C) Le mélange des deux azides précédemment obtenu de 5,75g est dissous dans 100ml d'isopropanol et chauffé à 65°. On ajoute par portions du Nickel de Raney (en maintenant la température) jusqu'à cessation de dégagement gazeux. On chauffe alors à 70° pendant 15min, refroidit à la température ambiante, filtre sur célite, lave celle-ci avec 100ml d'isopropanol, ajoute aux phases alcooliques 200ml d'HCl à 20% et évapore sous vide pour éliminer l'isopropanol, la phase aqueuse restante, refroidie, est neutralisée par NH₄OH. On extrait ensuite à l'éther (3x50ml), sèche (Na₂CO₃) et évapore le solvant sous vide pour isoler finalement un résidu huileux de 3,8g qui contient essentiellement deux amines primaires épimères (IR, GC/MS).
D) Le mélange d'amines précédent, 3,8g, est dissous dans 50ml d'acétonitrile contenant 4,3g de dibromo-1,5 pentane et 11g de K₂CO₃. Le mélange, fortement agité est porté au reflux pendant 72H puis refroidi à température ambiante. Après filtration, on ajoute 100ml d'HCl 20%, extrait à l'éther (2x50ml), les eaux acides, neutralisées par NH₄OH, sont à leur tour extraites à l'éther (3x50ml). Après séchage (Na₂CO₃), ces éthers sont évaporés sous vide pour donner un résidu huileux de 3,2g contenant essentiellement deux amines tertiaires isomères (IR, GC/MS). Le mélange obtenu est chromatographié sur alumine. L'éther de pétrole élue une première fraction d'huile claire, analytiquement pure, de 1,1g de c-méthyl-4-r-(pipéridino-1)-1(thiényl-2)-1 cyclohexane ; une deuxième fraction de cristaux blancs de 1,5g (68-69°), analytiquement pure du composé n^{o} 10 ou t-méthyl-2-r-(pipéridino-1)-1(thiényl-2)-1 cyclohexane et deux autres fractions de 300mg au total constituées d'amines primaires n'ayant pas réagi dont le mélange est recyclable lors d'une synthèse ultérieure. Par barbotage de HCl gazeux dans la solution éthérée des bases cristallisées, on fait précipiter leurs chlorhydrates, solides blancs qui, récupérés par essorage et séchés sous vide, fondent respectivement à 206-208° et 162-163° pour le composé n^{o} 10, HCl. Le rendement en composé n^{o} 10 base à partir de la cétone de départ est de 18,5%. Les deux structures stéréoisomères sont aisément différenciées par RMN du ¹³C (CDCl₃) des chlorhydrates (conformations anancomères) : dans le composé n^{o} 10, HCl, le méthyle, les carbones C2, C6 et C4 sont plus déblindés que dans l'isomère élué en tête, en raison de la position équatoriale du méthyle (au lieu d'axiale dans l'autre isomère).
Analyse centésimale du produit de formule brute C₆H₂₆NSCl :

| | trouvée | calculée |
|---|---|---|
| C | 63,88 | 64,11 |
| H | 8,64 | 8,68 |
| N | 4,64 | 4,68 |

### Exemple 15 : Préparation du t-hydroxy-2 phényl-1-r-(pipéridino-1)-1 cyclohexane de formule :

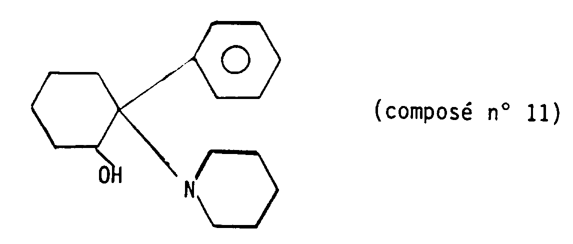

A) On prépare, dans 300ml d'éther anhydre, le réactif de Grignard résultant de l'action de 48,9g (0,3M) de bromobenzène sur 8,75g (0,35M) de magnésium en tournures. On y ajoute 19,6g (0,2M) de cyclohexanone dissous dans 300ml d'éther anhydre. On agite 3h au reflux, décompose le complexe par une solution saturée froide de NH₄Cl, puis, après décantation, on extrait les eaux à l'éther (3x200ml). Les éthers, séchés (Na₂SO₄), sont évaporés sous vide pour donner un solide jaunâtre qui, après deux cristallisations dans l'éther de pétrole, donne 25g de phényl-1 cyclohexanol blanc (70%) fondant à 60-61° (IR).
B) A 17,6g (0,1M) d'alcool fondu précédent on ajoute, sous agitation, 20ml d'un mélange d'acide sulfurique et d'acide acétique (20/80 v/v). Après 30sec., le mélange est jeté sur 100ml d'eau et de glace, puis extrait à l'éther (3x20ml) ; les éthers lavés à l'eau puis séchés (Na₂CO₃) sont évaporés sous vide et le liquide restant distillé sous pression réduite (0,5mmHg) pour donner une fraction (86-88°) de 11,8g (75%) de phényl-1 cyclohexène pur (IR).
C) On place 20g (0,125M) du phényl-1 cyclohexène précédent dans 200ml d'éther anhydre, on y ajoute, lentement, sous agitation, 26,6g (0,15M) d'acide m-chloroperbenzoïque dissous dans 200ml d'éther anhydre. On agite à 25° pendant 12h, puis ajoute une solution de NaOH à 10% en quantité suffisante pour neutraliser exactement l'acide mis en réaction. On décante, lave les éthers regroupés jusqu'à pH neutre, les sèche (Na₂SO₄) puis les évapore sous vide pour obtenir 20,8g d'époxyde brut. Cet époxyde est chromatographié sur alumine neutre, dans l'éther de pétrole contenant de l'éther (95/5 v/v), pour obtenir 17,8g d'une huile transparente (81%) analytiquement pure.
D) On dissout 10,5g (0,06M) d'époxyde et 30,8g (0,36M) de pipéridine dans 47g de méthanol. Après six jours de reflux sous agitation, on jette dans un mélange d'eau et de glace, extrait à l'éther (3x100ml), extrait ces derniers par HCl 20% (2x100ml) et neutralise la phase acide par NH₄OH. Les eaux sont extraites à l'éther (3x100ml), les éthers séchés (Na₂SO₄) sont évaporés sous vide à chaud pour donner 13,4g de solide jaunâtre. Ce dernier après plusieurs cristatlisations dans l'hexane donne 2,4g (15,4%) du composé n^{o} 11 sous la forme de solide blanc, analytiquement pur, fondant à 133-134°. Par barbotage de HCl gazeux dans la solution éthérée de ce composé, on fait précipiter son chlorhydrate, solide blanc qui, récupéré par essorage et séché sous vide, fond à 203-205°.
Analyse centésimale du produit de formule brute C₁₇H₂₅NO :

| | trouvée | calculée |
|---|---|---|
| C | 78,61 | 78,77 |
| H | 9,80 | 9,65 |
| N | 5,22 | 5,40 |

Dans le tableau 1 joint, on a regroupé les résultats des spectres de RMN du ¹³C des composés 1 à 11. Les spectres ont été enregistrés dans CDCl₃, à 20,147 MHz en ppm par rapport au tétraméthylsilane (TMS), les composés étant soit sous forme de base, soit sous forme de chlorhydrate (repérés par un astérisque).

### Exemple 16 : Test simple de la tige tournante.

Dans cet exemple, on teste les propriétés des composés n^{o} 1 à 5 et 8 à 11 sur des souris de race Swiss.

On administre aux souris par voie sous-cutanée différentes doses des composés à tester, puis on place les souris sur une tige tournante par lots de 10, isolées les unes des autres par des disques de séparation. Cette tige est en bois gratté et a un diamètre de 3cm et elle est animée horizontalement d'un mouvement circulaire uniforme autour de son axe à la vitesse de 10t/min.

On détermine la DE₅₀ qui est la dose pour laquelle on observe la chute de 50% des animaux d'expérience. Cette dose est exprimée en mg/kg.

Les résultats obtenus sont donnés dans le tableau 2 joint.

Au vu de ces résultats, on constate que tous les composés de l'invention sont actifs, puisque la DE₅₀ est inférieure à 25mg/kg à l'exception du composé n^{o} 8 qui présente une activité faible comparativement aux autres composés.

### Exemple 17 : Affinité des composés pour les sites de la PCP et de la TCP.

Dans cet essai, on détermine l'affinité de certains composés des exemples 5 à 15 par des expériences de compétition sur des homogénats de cerveaux de rats. Les ligands radioactifs utilisés sont (³H)PCP à une activité de 45Ci/mmol et (³H)TCP à une activité de 62Ci/mmol.

On incube une concentration fixe du ligand tritié (1nmol) en présence de concentrations croissantes du composé à tester et de l'homogénat (1mg de protéine par ml) en milieu tampon 50mM Hepes-Tris à pH 7,7, à 25°C. A l'équilibre, la concentration du composé testé empêchant la fixation de 50% du ligand tritié (IC50) reflète l'affinité de ce dernier pour le récepteur considéré.

Les résultats obtenus sont donnés dans le tableau 2 joint.

Au vu de ces résultats, on constate que les composés de l'invention sont très actifs car les concentrations nécessaires pour empêcher la fixation de 50% du ligand tritié sont très faibles.

### Exemple 18 : Test d'inhibition de la prise striatale synaptosomale de (³H)DA.

Ce test est effectué selon la méthode utilisée par Vignon et Lazdunski en opèrant de la façon suivante. Des rats Wistar pesant 200-250g sont tués par dislocation cervicale. Leurs cerveaux sont rapidement prélevés et les striata sont disséqués sur de la glace. Les striata sont ensuite homogénéisés avec un homogénéiseur Potter en verre Téflon dans 50 volumes de tampon Tris HCl 10mM contenant 0,32M de sucrose, à pH 7,4, et ils sont centrifugés à 1000g pendant 10min. Les surnageants sont centrifugés de plus à 10000g pendant 20min. Les culots résultants P₂ contenant les synaptosomes bruts sont utilisés sans purification supplémentaire.

On équilibre les synaptosomes avec le composé à tester pendant 30min à 30°C dans un milieu de Ringer (mM : 140NaCl, 5KCl, 2,6CaCl₂, 1,3MgSO₄, 10 Tris-HCl, pH = 7,4) + 200»M de pargyline.

On ajoute alors de la dopamine marquée (³H)DA à une concentration finale de 5nM pendant 5min à 30°C, et on interrompt la prise par filtration de l'incubat (150»l) sur des filtres GF/B. La prise non spécifique est mesurée à 4°C dans des incubations parallèles et est soustraite de la prise totale à 30°C. La IC50 est la concentration en composé qui empêche 50% de la capture spécifique par rapport au témoin en l'absence d'inhibiteur.

Les résultats obtenus sont donnés dans le tableau 2 joint.

### Exemple 19 : Test de compétition avec la 3H-QNB pour le récepteur muscarinique cholinergique cérébral.

Dans ces essais, on fait incuber une concentration fixe de ligand tritié (³H-QNB) en présence de concentrations variables des composés à tester et d'un homogénat de cerveau de rat à 1mg de protéine par ml environ.

La concentration de composé empêchant la fixation de 50% du ligand tritié (IC50) est une mesure de l'affinité de ce composé pour le récepteur muscarinique cholinergique cérébral. La fixation des ligands tritiés à l'homogénat est déterminée par la technique de filtration rapide sur les filtres de verre Whatman GF/B.

Les résultats obtenus sont donnés dans le tableau 2 joint.

Au vu de ces résultats, on constate que les composés testés ont pour la plupart très peu d'affinité pour le récepteur cholinergique muscarinique.

### Exemple 20 : Test de compétition avec la H³DHM pour le récepteur des opiacés.

Dans cet essai, on fait incuber une concentration fixe de morphine marquée ³H-DHM en présence de concentrations variables du composé testé et d'un homogénat de cerveau de rat. La concentration de composé empêchant la fixation de 50% de ³HDHM (IC50) est une mesure de l'affinité du composé pour le récepteur des opiacés. Le résultat obtenu est donné dans le tableau 2 joint.

Dans le tableau 2 joint, on a reporté également les résultats obtenus avec la PCP et la TCP, ainsi que les rapports H³DA/³H-TCP, ³HQNB/³H-TCP, et ³HDHM/³HTCP obtenus avec les différents composés testés.

Au vu de ce tableau, on constate que la sélectivité des composés de l'invention pour le site PCP par rapport aux sites DA, QNB et DHM est beaucoup plus importante que dans le cas de la PCP et de la TCP.

On remarque notamment que le rapport ³HDA/³H-TCP est supérieur à 100 avec les composés utilisés dans l'invention alors qu'il n'est respectivement que de 2,5 et 25,39 pour la PCP et la TCP.

### Exemple 21.

Cet exemple illustre l'effet des arylcyclohexylamines de l'invention sur des cultures "in vitro" de cellules de cerveaux de rats comme neuroprotecteur contre les atteintes aiguës provoquées par la présence de glutamate.

On prépare des cultures de neurones corticaux et hippocampaux à partir de foetus de rats âgés de 17 à 18 jours. Les hémisphères ou les hippocampes sont disséqués et immergés dans un tampon contenant de l'EDTA (Versène) pendant 10min. Les tissus sont ensuite dissociés mécaniquement dans une solution saline PUCK libre de calcium et de magnésium et centrifugés à 70g pendant 10min. Le culot de centrifugation est remis en suspension dans un milieu de culture constitué de 60% du milieu essentiel minimal de Eagle (MEM), 25% d'une solution saline de Hank, 5% de sérum humain, 5% de sérum de foetus de veau, 5% d'extrait d'embryon, qui sont complémentés avec 6% de glucose. On introduit les cellules à une densité de 10⁶ cellules par ml dans 24 puits Well revêtus préalablement avec de la polylysine D. On laisse croître les cultures pendant 3 semaines, 1/3 du milieu de culture étant renouvelé tous les 3 jours. On poursuit ensuite l'essai de la façon suivante. On ajoute du glutamate dissous dans 10»l de MEM au milieu afin d'atteindre une concentration finale en glutamate de 5.10⁻⁴M. Après une exposition de 5min, on change le milieu. Pour l'étude des dérivés antagonistes du glutamate, on ajoute au milieu de culture le composé à tester dans 10»l de MEM de façon à obtenir une concentration finale de 200, 20, ou 0,2.10⁻⁶M en composé. Cinq minutes plus tard, on ajoute du glutamate au milieu, puis on renouvelle le milieu après une exposition de 5min. On examine alors les cultures protégées et non protégées, et on les photographie à des intervalles allant de 30min à 24h.

Après 3 semaines de culture, les neurones corticaux apparaissent comme un mélange de cellules brillantes allant d'une taille petite à une taille moyenne où les noyaux et les nucléoles sont quelquefois brillants. Elles croissent au sommet d'une monocouche de cellules plates qui sont en majorité des astrocytes immunoréactifs à la protéine acide fibrillaire. Dans les cultures de neurones hippocampaux et de neurones corticaux, une exposition aiguë au glutamate a pour effet un gonflement rapide associé à une granularité augmentée des corps des cellules. Un tel phénomène est également observé dans les cellules témoins après un rapide lavage. Après 3h d'exposition au glutamate, les cellules se rétractent et une nécrose progressive des neurones est observée et 24h après il y a une perte maximale des neurones. Dans la plupart des cas, les seuls neurones survivants sont trouvés après 24h dans les cultures de cortex alors que les neurones hippocampaux sont complètement endommagés.

En revanche, lorsqu'on ajoute aux cultures 20»mol du composé n^{o}2 de l'invention, les cultures ne sont que très peu endommagées. Avec des quantités de 2»mol du composé n^{o} 2, on obtient seulement une protection limitée et avec 0,2»mol aucune protection comme l'indiquent les résultats du tableau 3 obtenus 24h après une exposition de 5min au glutamate. Cet effet protecteur est observé contre le gonflement aigu qui est moins intense et la nécrose qui est complètement bloquée. Après 24h, alors que la nécrose est maximale dans les cultures traitées au glutamate, les cultures de cellules hippocampales et corticales apparaissent saines comme les cultures témoins.

Ainsi, le composé de l'invention a un effet protecteur efficace sur les neurones hippocampaux et corticaux contre les dommages dûs au glutamate "in vitro".

Ces résultats confirment l'effet protecteur des antagonistes non compétitifs du NMDA.

En ce qui concerne le mécanisme mis en oeuvre pour cette protection, on suppose qu'un afflux massif de Ca²⁺ produit par l'activation des récepteurs NMDA est impliquée dans la mort des neurones.

Les composés utilisés dans l'invention atténuent ou bloquent l'afflux de Ca²⁺ et ses conséquences léthales. Cet effet protecteur doit de plus être probablement renforcé par la présence du glutamate libéré de façon exogène ou endogène, et les composés sont donc très efficaces pour le traitement des atteintes aiguës dûes à la présence du glutamate.

### Exemple 22.

Cet exemple illustre l'effet des arylcyclohexylamines de l'invention sur des cultures "in vitro" de neurones corticaux et hippocampaux, prélevés sur des foetus de rats âgés de 17 à 18 jours.

On prépare tout d'abord des cultures de neurones corticaux hippocampaux comme dans l'exemple 21 en introduisant les cellules à une densité de 10⁶ cellules/ml dans des puits et on laisse croître les cultures pendant des durées de 12 à 25 jours en renouvelant 1/3 du milieu de culture tous les 3 jours. On traite alors les cultures avec le composé à tester de façon à obtenir une concentration finale en composé de 20 »mol/litre. Après ce traitement, on apprécie la survie neuronale soit par examen direct sur les cellules vivantes en contraste de phase, soit après fixation et marquage immunocytochimique par des anticorps dirigés contre l'énolase neuronale.

Avec ce traitement, on observe une survie d'un grand nombre de neurones au-delà de deux mois de culture alors que généralement dans ce type de culture, la longévité des neurones ne dépasse guère 30 jours.

Le meilleur moment pour réaliser ce traitement se situe au début de la troisième semaine de culture qui correspond à la période à laquelle les neurons deviennent sensibles à la toxicité aigüe des acides aminés excitateurs naturellement présents dans le milieu de culture.

### Exemple 23.

Cet exemple illustre également l'effet des arylcyclohexylamines de l'invention sur la survie de culture de neurones "in vitro".

Dans cet exemple on prépare comme dans l'exemple 21 des cultures de neurones corticaux et hippocampaux. Au début de la troisième semaine de culture, on traite les cellules avec une dose unique du composé à tester et on poursuit la culture pendant 2 mois.

Après 2 mois de culture, les cellules qui ont été fixées et colorées pour mettre en évidence l'énolase spécifique des neurones, sont soumises à des comptages pour déterminer la quantité de cellules par unité de surface.

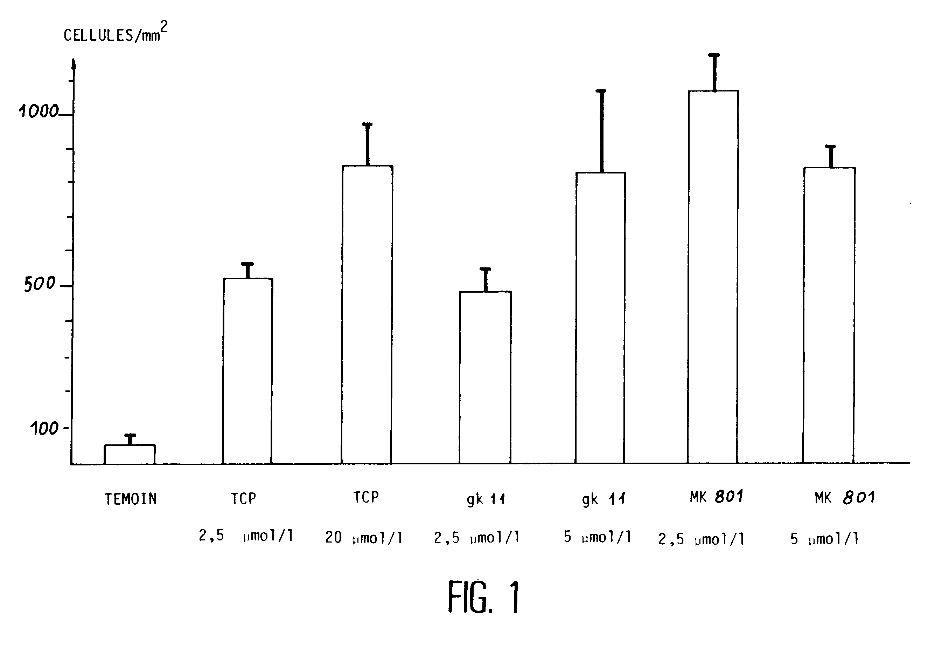

Les résultats obtenus sont donnés sur la figure 1 annexée qui est un histograme indiquant le nombre de cellules par mm² qui ont survécues après 2 mois de culture.

Les résultats sont donnés pour le témoin, c'est-à-dire pour des cultures non traitées, des cultures traitées par 2,5 »mol/l et 20 »mol/l de TCP, des cultures traitées par le composé n^{o} 9 à des concentrations de 2,5 »mol/l et 5 »mol/l et des cultures traitées par le compose MK801 à des concentrations de 2,5 »mol/l et 5 »mol/l. Le composé MK801 qui est connu pour avoir une activité en neuroprotection, est le (+)-méthyl-5-dihydro-10,11(5H) dibenzo(A,D) cycloheptèneimine-5,10 de formule 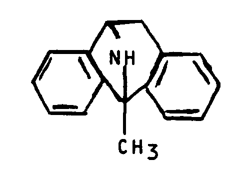
Sur la figure 1, il apparaît que les cultures témoins, après 2 mois de survie, contiennent en moyenne 50 neurones par mm². Les cultures traitées quel que soit le composé et la dose utilisés contiennent plus de 500 neurones par mm².

On a également effectué cette expérience avec des doses supérieures à celles mentionnées sur la figure 1, mais on n'a pas obtenu de résultats significativement différents.

On peut remarquer que le composé n^{o} 9 à la dose de 5 »mol/litre donne un résultat comparable à celui de la TCP à la dose de 20 »mol/l et à celui du MK801 à la dose de 5 »mol/l.

On peut conclure de cette expérience que la durée de vie de neurones cultivés "in vitro" peut être rallongée de façon considérable par un traitement unique avec des molécules qui sont des antagonistes non compétitifs du récepteur MMDA.

De plus, le composé n^{o} 9 de l'invention a une efficacité égale à celle du composé de référence MK801.

Les composés de l'invention sont donc très intéressants pour la lutte contre le vieillissement neuronal.

### Exemple 24.

Cet exemple illustre l'effet des arylcyclohexylamines sur le vieillissement cérébral.

On sait que le vieillissement cérébral quel qu'en soit la cause (vasculaire ou dégénérative) se traduit par une perte neuronale au niveau du cortex cérébral, de l'hippocampe, et à un moindre degré du cervelet. Cette perte cellulaire peut être quantifiée au moyen de techniques histologiques simples, et appréciée sur une plus grande échelle au moyen de techniques semi-automatiques d'analyse d'images.

Dans cet exemple, on a traité 6 souris âgées de 23 mois en injectant à trois souris par voie intrapéritonéale 5 mg/kg de TCP dans du soluté physiologique au 23ème, 24ème et 25ème mois et aux trois autres souris, également par voie intrapéritonéale, uniquement du soluté physiologique. Au 26ème mois, on a sacrifié toutes les souris par perfusion intracardiaque d'une solution de glutaraldéhyde à 5 %, on a coupé les cerveaux au vibratome et on a coloré les coupes par la technique de NISSL.

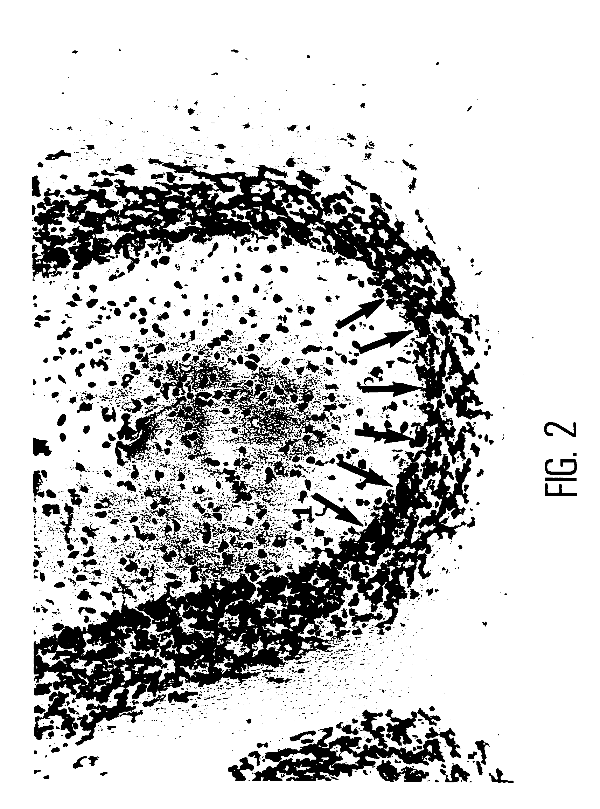

Les résultats obtenus sont donnés sur les figures 2 à 5 qui représentent les coupes du cerveau et du cervelet des souris traitées et des souris témoins non traitées.

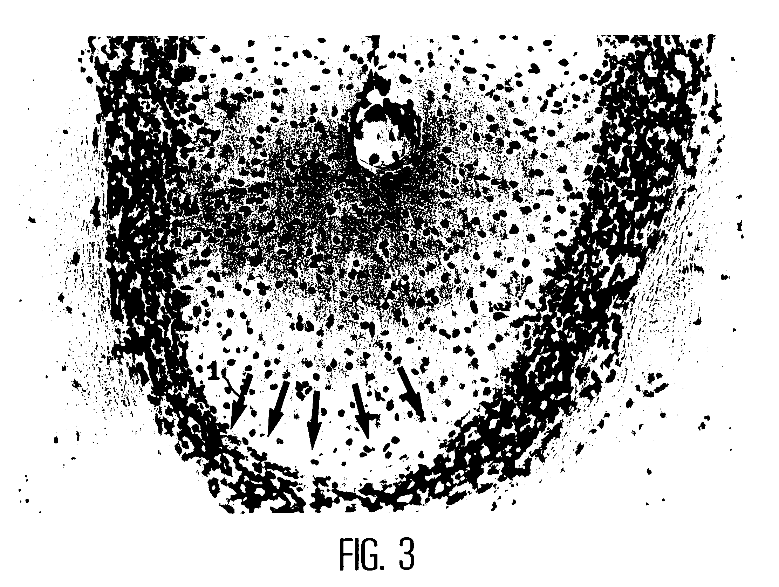

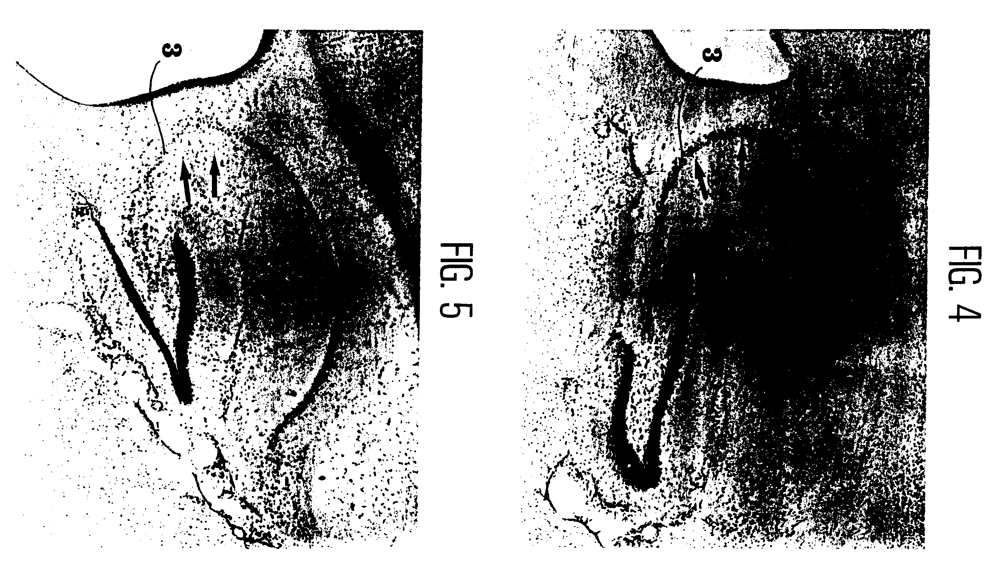

Sur la figure 3 on remarque au niveau du cervelet une raréfaction des cellules de Purkinjé (zone 1 repérée par les flèches) alors que sur la figure 2 on remarque que cette raréfaction dans la zone 1 est beaucoup moins importante. De la même façon, on observe sur la figure 5 une raréfaction des cellules pyramidales CA₃ de la corne d'Ammon, au niveau de l'hippocampe (zone 3 reprérée par la flèche), qui est beaucoup moins marquée dans la zone 3 de la figure 4 qui correspond au cas des souris traitées.

On a répété cett expérience en utilisant le composé n^{o}9 au lieu de la TCP et on a obtenue des résultats équivalents.

Ainsi, on constate que la TCP et les arylcyclohexylamines de l'invention sont efficaces pour lutter contre le vieillissement cérébral.

Les arylcyclohexyl amines de l'invention ont une faible toxicité. Ainsi la DL50 par voie sous cutanée chez le rat est supérieure à 60mg/kg pour les composés n^{o} 3 et 4.

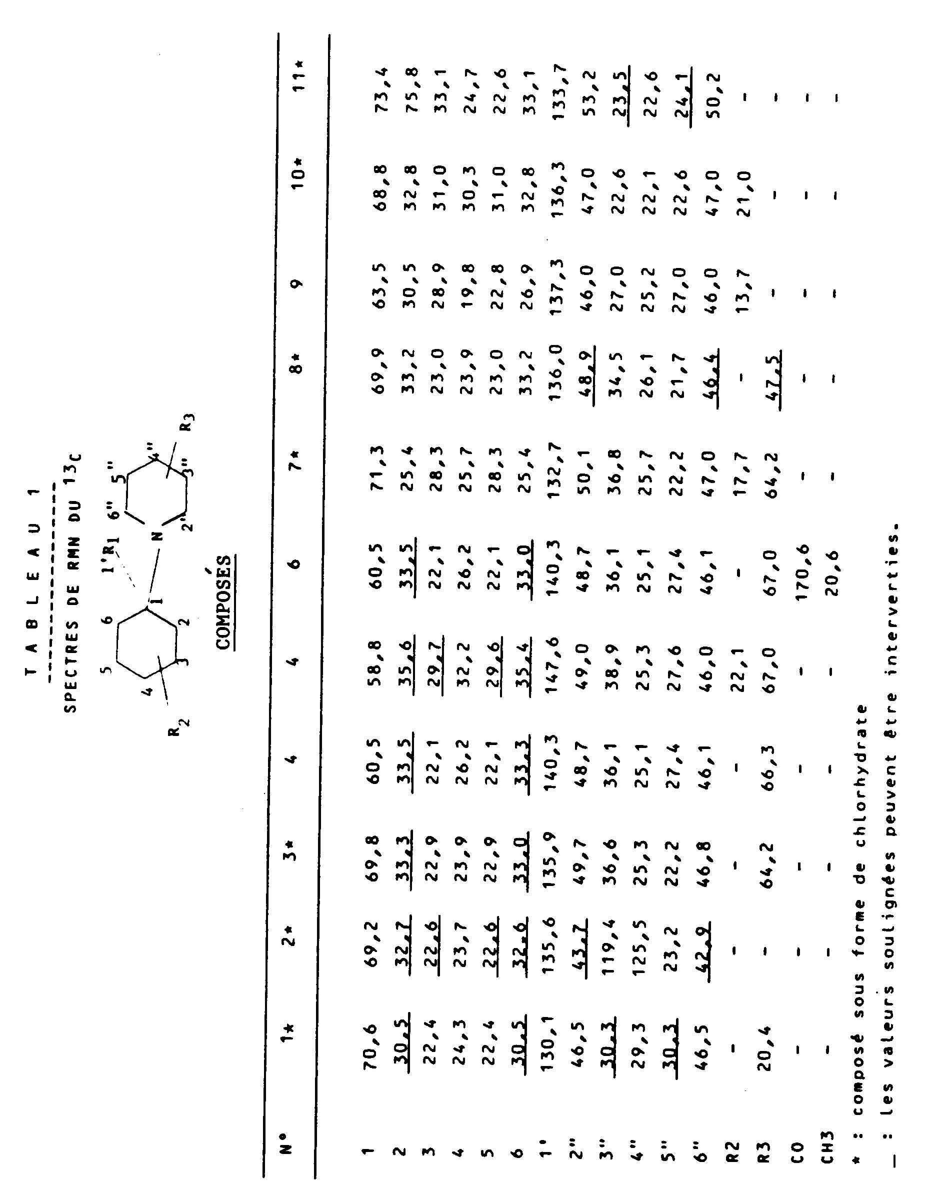

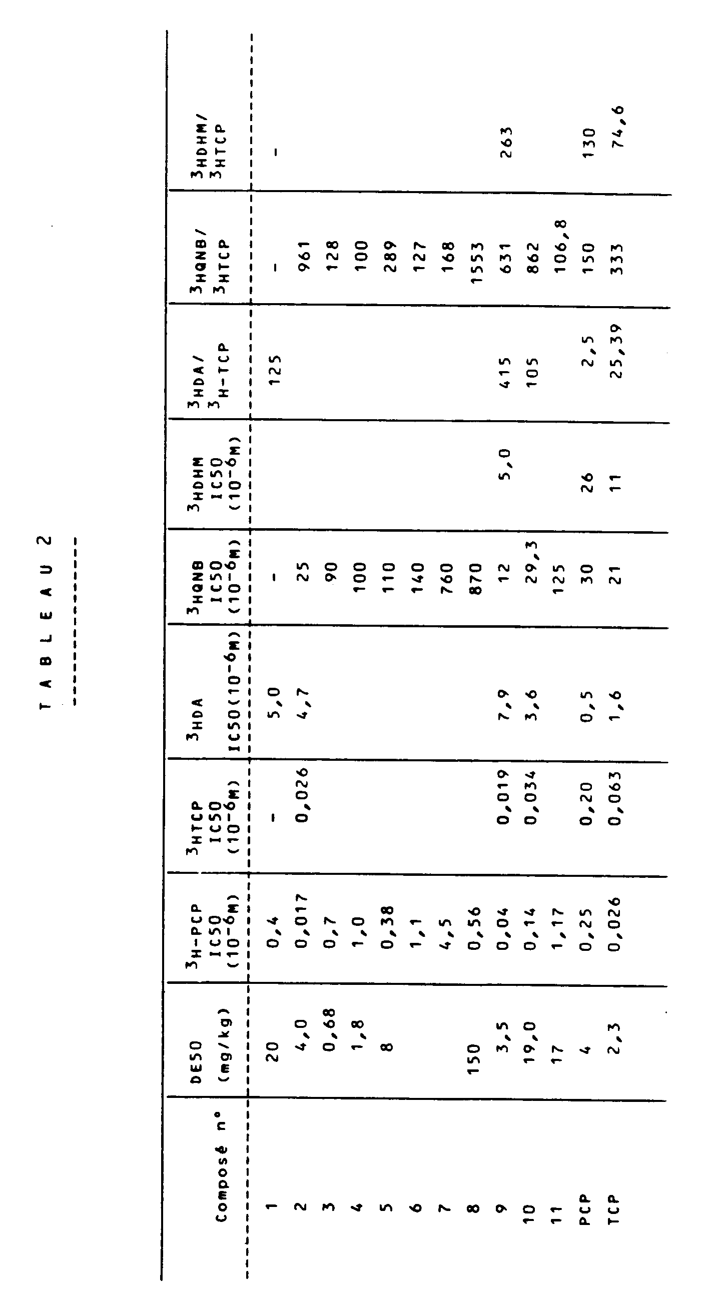

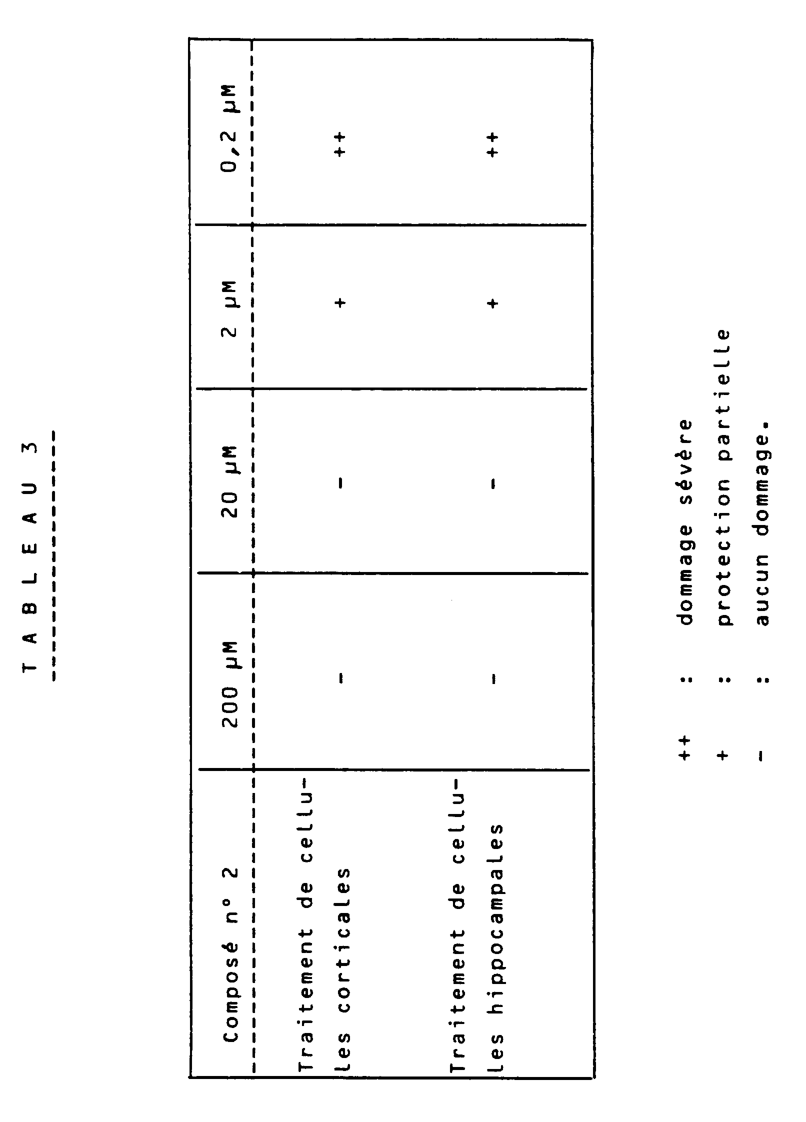

## Revendications

1. Utilisation d'une arylcyclohexylamine répondant aux formules : 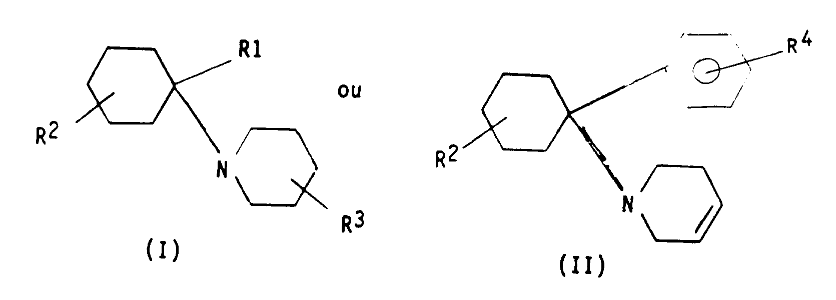 dans lesquelles :
R¹ représente 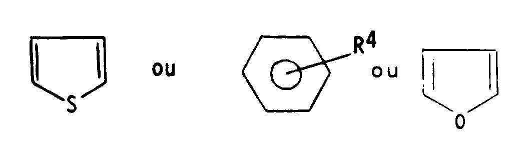 avec R⁴ étant un atome d'hydrogène, un atome de fluor, un atome d'iode ou un radical méthyle,
R² représente un atome d'hydrogène, le radical OH ou le radical CH₃, et
R³ représente un atome d'hydrogène ou le radical CH₂R⁵ avec R⁵ représentant H, OH, Cl, Br ou CH₃COO, à condition que R² et R³ ne soient pas tous deux un atome d'hydrogène, ou d'un sel d'addition à un acide pharmaceutiquement acceptable de cette arylcyclohexylamine, pour la fabrication d'un médicament neuroprotecteur destiné à empêcher les atteintes cérébrales pouvant se produire lors d'affections aigües et à protéger le cerveau contre le vieillissement.

2. Utilisation selon la revendication 1, caractérisée en ce que l'arylcyclohexylamine répond à la formule : 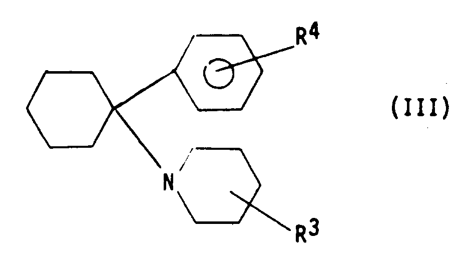 dans laquelle :
R³ représente le radical méthyle ou le radical CH₂R⁵ avec R⁵ représentant OH ou CH₃COO, et
R⁴ représente un atome d'hydrogène.

3. Utilisation selon la revendication 1, caractérise en ce que l'arylcyclohexylamine répond à la formule : 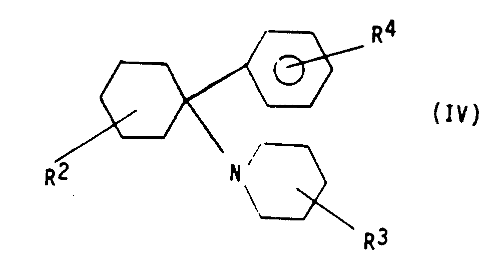 dans laquelle :
R² est le radical méthyle,
R³ est le radical CH₂R⁵ avec R⁵ représentant OH, et
R⁴ est un atome de fluor.

4. Utilisation selon la revendication 1, caractérisé en ce que l'arylcyclohexylamine répond à la formule :

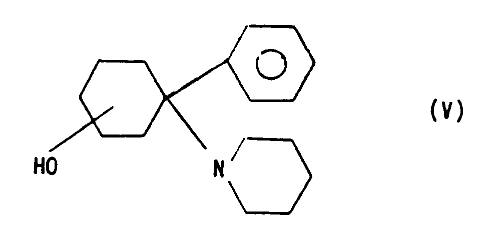

5. Utilisation selon la revendication 1, caractérisée en ce que l'arylcyclohexylamine répond à la formule : 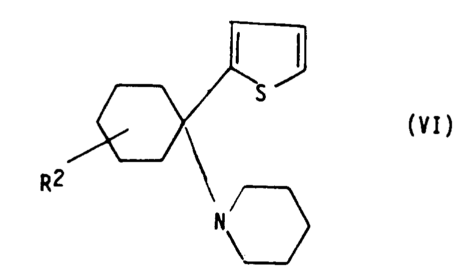 dans laquelle R² est le radical méthyle.

6. Utilisation selon la revendication 1, caractérisée en ce que l'arylcyclohexylamine répond à la formule : 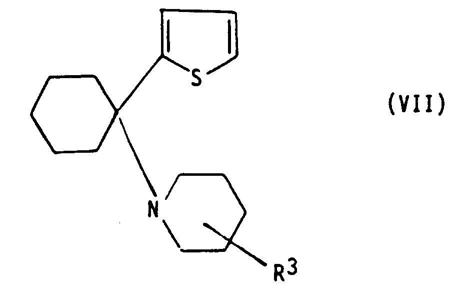 dans laquelle R³ est le radical CH₂R⁵ avec R⁵ représentant OH ou Cl.

7. Utilisation selon la revendication 1, caractérisée en ce que l'arylcyclohexylamine répond à la formule : 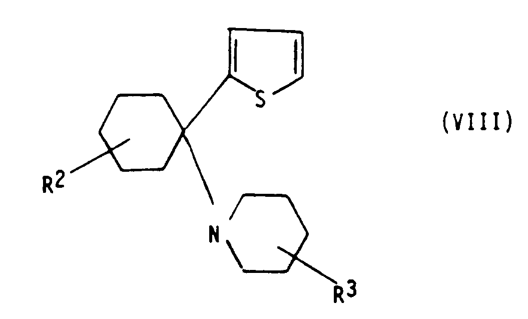 dans laquelle R² est le radical méthyle et R³ est le radical CH₂OH.

8. Utilisation selon la revendication 1, caractérisée en ce que l'arylcclohexylamine répond à la formule : 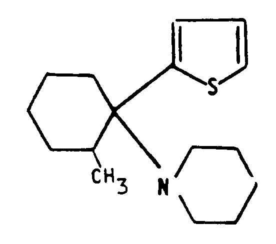 dans laquelle CH₃ est en position cis par rapport à la pipéridine.

9. Utilisation selon l'une quelconque des revendications 1, 3, 4, 5 et 7, caractérisée en ce que R² est en position ortho.

## Claims

1. Use of an arylcyclohexylamine in accordance with the formulas: 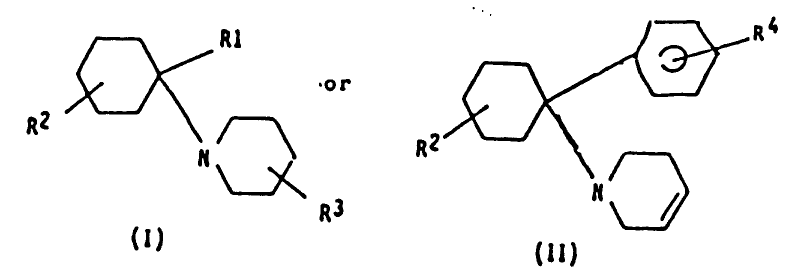 in which:
R¹ represents 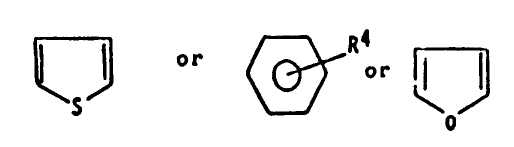 with R⁴ being a hydrogen atom, a fluorine atom, an iodine atom or a methyl radical, R² a hydrogen atom, the OH radical or the CH₃ radical and R³ a hydrogen atom or the radical CH₂R⁵ with R⁵ representing H, OH, Cℓ, Br or CH₃COO, provided that R² and R³ are not both a hydrogen atom, or an addition salt to a pharmaceutically acceptable acid of said arylcyclohexylamine, for the preparation of a neuroprotective medicament intended to prevent cerebral attacks which can occur during acute ailments or diseases and protect the brain against aging.

2. Use according to claim 1, characterized in that the arylcyclohexylamine is in accordance with the formula: 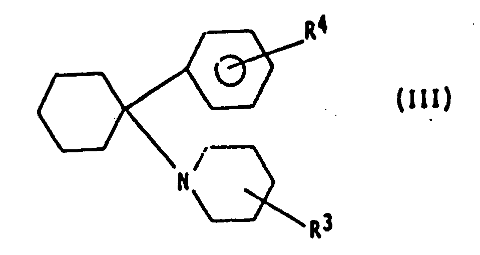 in which R³ stands for the methyl radical or the radical CH₂R⁵ with R⁵ representing OH or CH₃COO and R⁴ stands for a hydrogen atom.

3. Use according to claim 1, characterized in that the arylcyclohexylamine is in accordance with the formula: 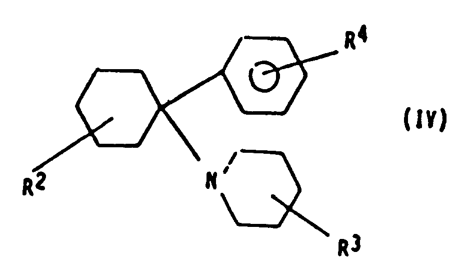 in which R² is the methyl radical, R³ the radical CH₂R⁵ with R⁵ representing OH and R⁴ is a fluorine atom.

4. Use according to claim 1, characterized in that the arylcyclohexylamine is in accordance with formula:

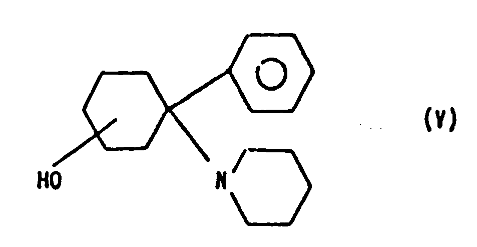

5. Use according to claim 1, characterized in that the arylcyclohexylamine is in accordance with formula: 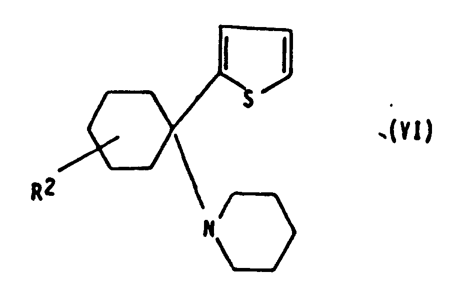 in which R² is the methyl radical.

6. Use according to claim 1, characterized in that the arylcyclohexylamine is in accordance with the formula: 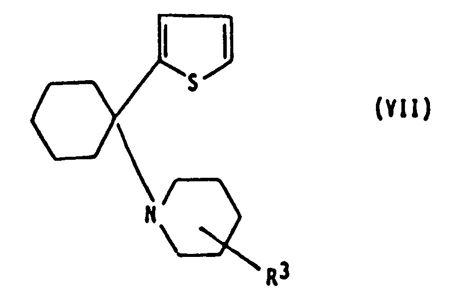 in which R³ is the CH₂R⁵ radical with R⁵ representing OH or Cℓ.

7. Use according to claim 1, characterized in that the arylcyclohexylamine is in accordance with the formula: 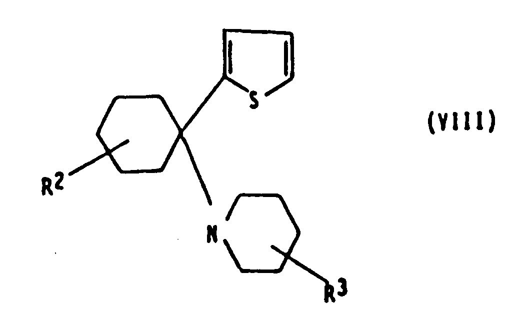 in which R² is the methyl radical and R³ the radical CH₂OH.

8. Use according to claim 1, characterized in that the arylcyclohexylamine is in accordance with the formula: 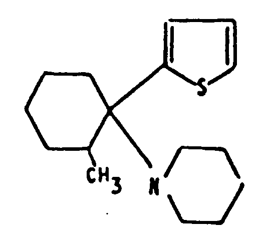 in which CH₃ is in the cis position with respect to the piperidine.

9. Use according to any one of the claims 1, 3, 4, 5 and 7, characterized in that R² is in the ortho position.

## Patentansprüche

1. Verwendung eines Arylcyclohexylamins entsprechend den Formeln: 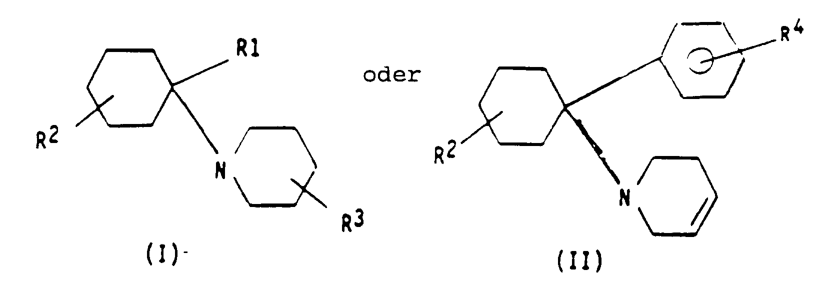 in welchen:
R¹ 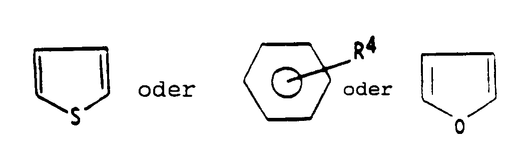 bedeutet, wobei R⁴ ein Wasserstoffatom, ein Fluoratom, ein Iodatom oder ein Methylrest ist,
R² ein Wasserstoffatom, den Rest OH oder den Rest CH₃ bedeutet, und
R³ ein Wasserstoffatom oder den Rest CH₂R⁵ bedeutet, wobei R⁵ H, OH, Cl, Br oder CH₃COO bedeutet, vorausgesetzt, daß R² und R³ nicht beide ein Wasserstoffatom sind, oder eines pharmazeutisch annehmbaren Säureadditionssalzes dieses Arylcyclohexylamins zur Herstellung eines die Nerven schützenden Arzneimittels, welches dazu bestimmt ist, zerebrale Schädigungen zu verhindern, die sich bei akuten krankhaften Zuständen einstellen können, und das Gehirn gegen das Altern zu schützen.

2. Verwendung nach Anspruch 1**, dadurch gekennzeichnet**, daß das Arylcyclohexylamin der Formel entspricht: 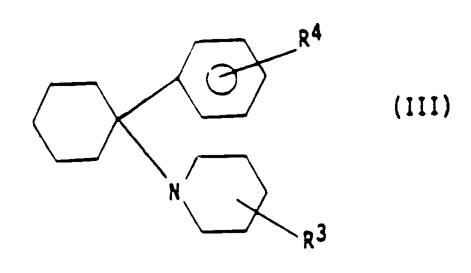 in welcher:
R³ den Methylrest oder den Rest CH₂R⁵ bedeutet, wobei R⁵ OH oder CH₃COO bedeutet, und
R⁴ ein Wasserstoffatom bedeutet.

3. Verwendung nach Anspruch 1, **dadurch gekennzeichnet**, daß das Arylcyclohexylamin der Formel entspricht: 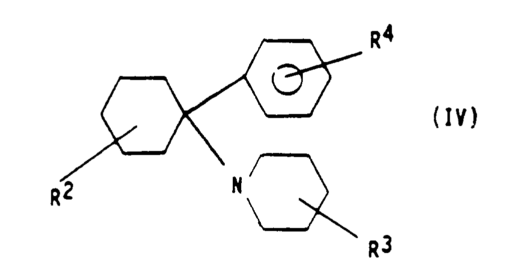 in welcher:
R² der Methylrest ist,
R³ der Rest CH₂R⁵ ist, wobei R⁵ OH bedeutet, und
R⁴ ein Fluoratom ist.

4. Verwendung nach Anspruch 1, **dadurch gekennzeichnet**, daß das Arylcyclohexylamin der Formel entspricht:

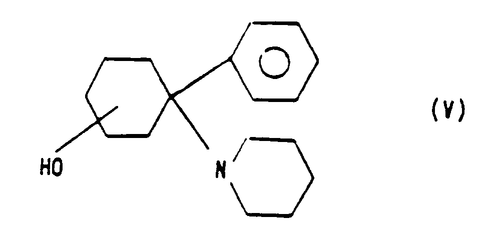

5. Verwendung nach Anspruch 1, **dadurch gekennzeichnet**, daß das Arylcyclohexylamin der Formel entspricht: 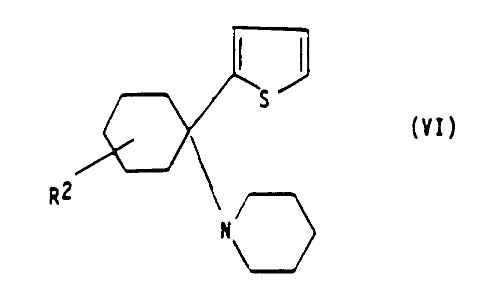 in welcher R² der Methylrest ist.

6. Verwendung nach Anspruch 1, **dadurch gekennzeichnet**, daß das Arylcyclohexylamin der Formel entspricht: 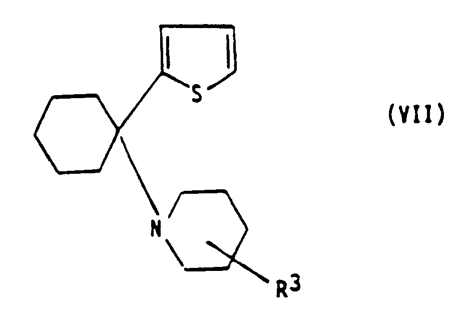 in welcher R³ der Rest CH₂R⁵ ist, wobei R⁵ OH oder Cl bedeutet.

7. Verwendung nach Anspruch 1, **dadurch gekennzeichnet**, daß das Arylcyclohexylamin der Formel entspricht: 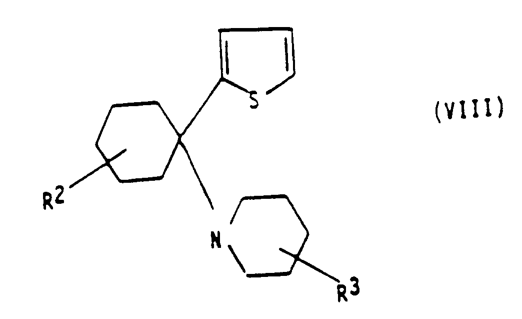 in welcher R² der Methylrest ist und R³ der Rest CH₂OH ist.

8. Verwendung nach Anspruch 1, **dadurch gekennzeichnet**, daß das Arylcyclohexylamin der Formel entspricht: 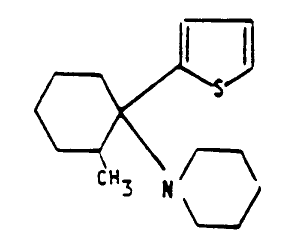 in welcher CH₃ in cis-Stellung bezüglich des Piperidins ist.

9. Verwendung nach einem der Ansprüche 1, 3, 4, 5 und 7, **dadurch gekennzeichnet**, daß R² in ortho-Stellung ist.
